(19) Europäisches Patentamt
European Patent Office
Office européen des brevets

(11) EP 0 718 287 A2

(12) **EUROPÄISCHE PATENTANMELDUNG**

(43) Veröffentlichungstag:
**26.06.1996 Patentblatt 1996/26**

(51) Int. Cl.[6]: **C07D 213/74**, A61K 31/415

(21) Anmeldenummer: **95120118.5**

(22) Anmeldetag: **19.12.1995**

(84) Benannte Vertragsstaaten:
**AT BE CH DE DK ES FR GB GR IE IT LI LU MC NL PT SE**

(30) Priorität: **23.12.1994 DE 4446300**
**08.09.1995 DE 19533224**

(71) Anmelder: **Dr. Karl Thomae GmbH**
**D-88397 Biberach (DE)**

(72) Erfinder:
- **Pieper, Helmut, Dr. Dipl.-Ing.**
**D-88400 Biberach (DE)**
- **Austel, Volkhard, Prof. Dr. Dipl.-Chem.**
**D-88400 Biberach (DE)**
- **Himmelsbach, Frank, Dr. Dipl.-Chem.**
**D-8441 Mittelbiberach (DE)**
- **Linz, Günther, Dr. Dipl.-Chem.**
**D-88441 Mittelbiberach (DE)**
- **Guth, Brian, Dr.**
**D-88447 Warthausen (DE)**
- **Weisenberger, Johannes, Dr. Dipl.-Chem.**
**D-884400 Biberach (DE)**

(54) **Piperazinderivate, diese Verbindungen enthaltende Arzneimittel, deren Verwendungen und Verfahren zu ihrer Herstellung**

(57) Die vorliegende Erfindung betrifft Piperazinderivate der allgemeinen Formel

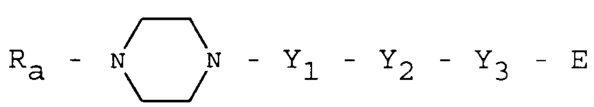

, (I)

in der
$R_a$, $Y_1$ bis $Y_3$ und E wie Anspruch 1 definiert sind, deren Tautomere, deren Stereoisomere, einschließlich ihrer Gemische, und deren Salze, insbesondere deren Salze mit physiologisch verträglichen Säuren oder Basen, welche wertvolle pharmakologische Eigenschaften aufweisen, vorzugsweise aggregationshemmende Wirkungen, diese Verbindungen enthaltende Arzneimittel und deren Verwendung sowie Verfahren zu ihrer Herstellung.

EP 0 718 287 A2

Printed by Rank Xerox (UK) Business Services
2.12.4/3.4

## Beschreibung

Die vorliegende Erfindung betrifft Piperazinderivate der allgemeinen Formel

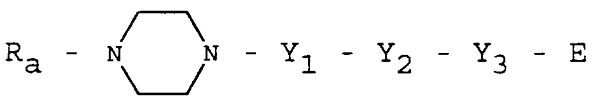

, (I)

deren Tautomere, deren Stereoisomere, einschließlich ihrer Gemische, und deren Salze, insbesondere deren Salze mit physiologisch verträglichen Säuren oder Basen, welche wertvolle pharmakologische Eigenschaften aufweisen, vorzugsweise aggregationshemmende Wirkungen, diese Verbindungen enthaltende Arzneimittel und deren Verwendung sowie Verfahren zu ihrer Herstellung.

In der obigen allgemeinen Formel I bedeutet
mit der Maßgabe, daß, wenn $R_a$ eine 4-Pyridylgruppe darstellt, nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO$-Gruppe,
$Y_2$ eine 1,3- oder 1,4-Phenylengruppe,
$Y_3$ eine-$CH_2CO-$, $-CH_2CH_2CO-$ oder $-OCH_2CO$-Gruppe und
E eine Hydroxy-, Methoxy- oder Ethoxygruppe oder

(b) $Y_1$ eine $-CH_2CO$-Gruppe,
$Y_2$ eine 3- oder 4-Piperidinylengruppe,
$Y_3$ eine -CO-, $-CH_2CO-$ oder $-OCH_2CO$-Gruppe und
E eine Hydroxy- oder Ethoxygruppe darstellen,

(c) $Y_1$ eine $-COCH_2$-Gruppe,
$Y_2$ eine 1,4-Phenylengruppe,
$Y_3$ eine $-OCH_2CO$-Gruppe und
E eine Hydroxy- oder tert.Butyloxygruppe darstellen,

$R_a$ eine Pyridylgruppe,
$Y_1$ eine -CO-, -CO-CO-, $-A_1$-CO-, -CO-$A_1$-, $-SO_2-A_2-$, $-A_2-SO_2-$, -CO-$A_1$-CO-, -CO-$NR_1$-CO-, -CO-$NR_1$-$A_2$-, -CO-$NR_1$-$A_2$-CO- oder -CO-$A_2$-$NR_1$-CO-Gruppe, in denen

$R_1$ ein Wasserstoffatom, eine $C_{1-5}$-Alkyl-, Aryl- oder Aryl-$C_{1-3}$-alkylgruppe,

$A_1$ eine gegebenenfalls durch eine $C_{1-5}$-Alkyl-, Cyclohexyl-$C_{1-3}$-alkyl-, Aryl- oder Aryl-$C_{1-3}$-alkylgruppe oder auch durch eine $R_1O$-Gruppe, sofern diese nicht in $\alpha$-Stellung zu einem Stickstoffatom steht, substituierte n-$C_{1-5}$-Alkylengruppe und

$A_2$ eine gegebenenfalls durch eine $C_{1-5}$-Alkyl-, Aryl- oder Aryl-$C_{1-3}$-alkylgruppe substituierte n-$C_{1-4}$-Alkylengruppe darstellen,

$Y_2$ eine Phenylen-, Cyclohexylen- oder Pyridinylengruppe, eine 3-Piperidinylen-, 4-Piperidinylen- oder 1,4-Piperazinylengruppe, in denen jeweils eine oder zwei zu einem Stickstoffatom benachbarte Methylengruppen durch eine Carbonylgruppe ersetzt sein können, eine -$NR_1$-B- oder -O-B-Gruppe, wobei die Verknüpfung mit der $Y_1$-Gruppe über das Stickstoffatom der -$NR_1$-Gruppe oder über das Sauerstoffatom der -O-B-Gruppe erfolgt, in denen

$R_1$ wie eingangs definiert ist und

B eine Phenylen-, Cyclohexylen-, Piperidinylen- oder Pyridinylengruppe darstellt, wobei die Verknüpfung der Piperidinylengruppe jeweils über die 3- oder 4-Stellung mit dem Rest -$NR_1$- oder mit dem Sauerstoffatom erfolgt und in der zusätzlich eine zu einem Stickstoffatom benachbarte Methylengruppe durch eine Carbonylgruppe ersetzt sein kann,

$Y_3$ eine -CO-, $-A_2$-CO-, $-CH_2-CH(NHR_2)-CO-$, $-NR_2-A_3-CO-$, $-O-A_3-CO-$ oder $-CO-A_3-CO$-Gruppe, in denen

$R_1$ und $A_2$ wie eingangs definiert sind,

$A_3$ eine gegebenenfalls durch eine $C_{1-5}$-Alkyl-, Aryl- oder Aryl-$C_{1-3}$-alkylgruppe substituierte n-$C_{1-3}$-Alkylengruppe und

$R_2$ ein Wasserstoffatom, eine $C_{1-5}$-Alkyl-, Aryl-$C_{1-3}$-alkyl-, Aryl-, $C_{1-5}$-Alkoxycarbonyl-, $C_{1-5}$-Alkanoyl-, $C_{1-5}$-Alkylsulfonyl-, Aryl-$C_{1-3}$-alkylsulfonyl- oder Arylsulfonylgruppe, eine gegebenenfalls durch eine Aryl- oder Aryl-$C_{1-3}$-alkylgruppe substituierte Formylgruppe darstellen sowie die Verknüpfung der $-A_2$-CO-Gruppe über den Rest $A_2$, der -$NR_2$-$A_3$-CO-Gruppe über die -$NR_2$-Gruppe und der -O-$A_3$-CO-Gruppe über das Sauerstoffatom mit dem Rest $Y_2$ erfolgt,

wobei jedoch eine $-NR_2-$ oder $-O-A_3-CO-$Gruppe nicht mit einem Stickstoffatom des Restes $Y_2$ verknüpft sein kann, und E eine Hydroxygruppe, eine Alkoxygruppe mit 1 bis 6 Kohlenstoffatomen, eine Phenylalkoxygruppe, in der der Alkoxyteil 1 bis 3 Kohlenstoffatome enthalten kann, eine Cycloalkoxygruppe mit 3 bis 9 Kohlenstoffatomen, in welcher der Cycloalkylteil mit 5 bis 8 Kohlenstoffatomen zusätzlich durch ein oder zwei Alkylgruppen mit jeweils 1 bis 3 Kohlenstoffatomen substituiert sein kann, eine Cycloalkoxygruppe mit 5 bis 8 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe, in denen der Alkyl- und Alkoxyteil jeweils 1 bis 3 Kohlenstoffatomen enthalten kann, oder durch eine Alkanoylgruppe mit 2 bis 6 Kohlenstoffatomen substituierte Iminogruppe ersetzt ist und der Cycloalkylteil zusätzlich durch ein oder zwei Alkylgruppen mit jeweils 1 bis 3 Kohlenstoffatomen substituiert sein kann, eine Cycloalkenyloxygruppe, in der der Cycloalkenylteil 4 bis 7 Kohlenstoffatome enthalten kann, eine Alkenyloxy-, Phenylalkenyloxy-, Alkinyloxy- oder Phenylalkinyloxygruppe mit der Maßgabe, daß keine Bindung an das Sauerstoffatom von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkoxygruppe, in der der Cycloalkylteil 3 bis 8 Kohlenstoffatome und der Alkoxyteil 1 bis 3 Kohlenstoffatome enthalten kann, eine Bicycloalkoxygruppe mit insgesamt 8 bis 10 Kohlenstoffatomen, die im Bicycloalkylteil zusätzlich durch ein oder zwei Alkylgruppen mit jeweils 1 bis 3 Kohlenstoffatomen substituiert sein kann, eine 1,3-Dihydro-3-oxo-1-isobenzfuranyloxygruppe oder eine $R_5-CO-O-(R_3CR_4)-O-$Gruppe, in der

$R_3$ ein Wasserstoffatom, eine $C_{1-6}$-Alkyl-, $C_{3-7}$-Cycloalkyl- oder Phenylgruppe,
$R_4$ ein Wasserstoffatom oder eine $C_{1-6}$-Alkylgruppe und
$R_5$ eine $C_{1-5}$-Alkyl-, $C_{1-5}$-Alkoxy-, $C_{5-7}$-Cycloalkyl- oder $C_{5-7}$-Cycloalkoxygruppe darstellen,

oder E eine $\alpha$-Aminogruppe einer natürlichen Aminosäure und deren Ester.

Unter dem bei der Definition der vorstehenden Resten erwähnten Ausdrücken "eine Arylgruppe," "eine Phenylgruppe" oder "eine Phenylengruppe" ist jeweils insbesondere eine gegebenenfalls durch Fluor-, Chlor-, Brom- oder Jodatome, durch Alkyl-, Trifluormethyl-, Nitro-, Amino-, Alkylamino-, Dialkylamino-, Alkanoylamino-, Hydroxy-, Alkoxy-, Carboxy-, Alkoxycarbonyl-, Hydroxycarbonylalkoxy-, Alkoxycarbonylalkoxy-, Aminocarbonyl-, Alkylaminocarbonyl- oder Dialkylaminocarbonylgruppen mono-, di- oder trisubstituierte Phenyl- oder Phenylengruppe, wobei die Substituenten gleich oder verschieden sein können und die vorstehend erwähnten Alkyl- und Alkoxyteile jeweils 1 bis 3 Kohlenstoffatome enthalten können, und

unter den Estern einer natürlichen $\alpha$-Aminogruppe deren $C_{1-6}$-Alkyl-, $C_{2-6}$-Alkenyl-, $C_{5-7}$-Cycloalkyl-, Phenyl- oder Phenyl-$C_{1-3}$-alkylester wie der Methyl-, Ethyl-, n-Propyl-, Isopropyl-, tert.Butyl-, Allyl-, Phenyl- oder Benzylester zu verstehen.

Bevorzugte Verbindungen der obigen allgemeinen Formel I sind diejenigen, in denen
mit der Maßgabe, daß, wenn $R_a$ eine 4-Pyridylgruppe darstellt, nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO-$Gruppe,
$Y_2$ eine 1,3- oder 1,4-Phenylengruppe,
$Y_3$ eine-$CH_2CO-$, $-CH_2CH_2CO-$ oder $-OCH_2CO-$Gruppe und
E eine Hydroxy-, Methoxy- oder Ethoxygruppe oder

(b) $Y_1$ eine $-CH_2CO-$Gruppe,
$Y_2$ eine 3- oder 4-Piperidinylengruppe,
$Y_3$ eine $-CO-$, $-CH_2CO-$ oder $-OCH_2CO-$Gruppe und
E eine Hydroxy- oder Ethoxygruppe darstellen,

(c) $Y_1$ eine $-COCH_2-$Gruppe,
$Y_2$ eine 1,4-Phenylengruppe,
$Y_3$ eine $-OCH_2CO-$Gruppe und
E eine Hydroxy- oder tert.Butyloxygruppe darstellen,

$R_a$ eine 3- oder 4-Pyridylgruppe,
$Y_1$ eine $-CO-$, $-CO-CO-$, $-A_1-CO-$, $-CO-A_1-$ oder $-CO-A_1-CO-$Gruppe, in denen

$A_1$ eine gegebenenfalls durch eine $C_{1-5}$-Alkyl-, Phenyl- oder Phenyl-$C_{1-3}$-alkylgruppe substituierte n-$C_{1-5}$-Alkylengruppe darstellt, wobei die Phenylkerne der vorstehend erwähnten Phenyl- und Phenylalkylgruppen jeweils zusätzlich durch eine Hydroxy- oder Methoxygruppe substituiert sein können,

$Y_2$ eine 1,3-Phenylen-, 1,4-Phenylen-, 3-Piperidinylen-, 4-Piperidinylen-, 1,4-Piperazinylen- oder $-NR_1-B-$Gruppe, wobei die Verknüpfung mit der $Y_1$-Gruppe über das Stickstoffatom der $-NR_1-$Gruppe erfolgt und

$R_1$ ein Wasserstoffatom, eine $C_{1-5}$-Alkyl-, Phenyl- oder Phenyl-$C_{1-3}$-alkylgruppe und
B eine 1,3-Phenylen-, 1,4-Phenylen-, 1,3-Cyclohexylen-, 1,4-Cyclohexylen-, 3-Piperidinylen- oder 4-Piperidinylengruppe darstellen,

$Y_3$ eine $-CO-$, $-A_2-CO-$, $-NR_2-A_3-CO-$ oder $-O-A_3-CO-$Gruppe, in denen

$A_2$ eine gegebenenfalls durch eine $C_{1-5}$-Alkyl-, Phenyl- oder Phenyl-$C_{1-3}$-alkylgruppe substituierte n-$C_{1-4}$-Alkylengruppe,

$A_3$ eine gegebenenfalls durch eine $C_{1-5}$-Alkyl-, Phenyl- oder Phenyl-$C_{1-3}$-alkylgruppe substituierte n-$C_{1-3}$-Alkylengruppe und

$R_2$ ein Wasserstoffatom, eine $C_{1-5}$-Alkyl-, Phenyl-$C_{1-3}$-alkyl-, Phenyl-, $C_{1-5}$-Alkoxycarbonyl- oder $C_{1-5}$-Alkanoylgruppe darstellen sowie die Verknüpfung der -$A_2$-CO-Gruppe über den Rest $A_2$, der -$NR_2$-$A_3$-CO-Gruppe über die -$NR_2$-Gruppe und der -O-$A_3$-CO-Gruppe über das Sauerstoffatom mit dem Rest $Y_2$ erfolgt, wobei jedoch eine -$NR_2$- oder -O-$A_3$-CO-Gruppe nicht mit einem Stickstoffatom des Restes $Y_2$ verknüpft sein kann,

und E eine Hydroxygruppe, eine Alkoxygruppe mit 1 bis 6 Kohlenstoffatomen, eine Phenylalkoxygruppe, in der der Alkoxyteil 1 bis 3 Kohlenstoffatome enthalten kann, eine Cycloalkoxygruppe mit 4 bis 7 Kohlenstoffatomen oder eine $R_5$-CO-O-($R_3CR_4$)-O-Gruppe, in der

$R_3$ ein Wasserstoffatom, eine $C_{1-6}$-Alkyl-, $C_{3-7}$-Cycloalkyl- oder Phenylgruppe,

$R_4$ ein Wasserstoffatom oder eine $C_{1-6}$-Alkylgruppe und

$R_5$ eine $C_{1-5}$-Alkyl-, $C_{1-5}$-Alkoxy-, $C_{5-7}$-Cycloalkyl- oder $C_{5-7}$-Cycloalkoxygruppe darstellen,

oder E eine α-Aminogruppe einer natürlichen Aminosäure oder deren Estern bedeuten,

deren Tautomere, deren Stereoisomere, einschließlich ihrer Gemische, und deren Salze.

Besonders bevorzugte Verbindungen der obigen allgemeinen Formel I sind diejenigen, in denen mit der Maßgabe, daß nicht gleichzeitig

(a) $Y_1$ eine -$CH_2$CO-, -CH($CH_3$)CO-, -C($CH_3$)$_2$CO-, -$CH_2CH_2$CO- oder -$CH_2$CH($CH_3$)CO-Gruppe,
$Y_2$ eine 1,3- oder 1,4-Phenylengruppe,
$Y_3$ eine-$CH_2$CO-, -$CH_2CH_2$CO- oder -O$CH_2$CO-Gruppe und
E eine Hydroxy-, Methoxy- oder Ethoxygruppe oder

(b) $Y_1$ eine -$CH_2$CO-Gruppe,
$Y_2$ eine 3- oder 4-Piperidinylengruppe,
$Y_3$ eine -CO-, -$CH_2$CO- oder -O$CH_2$CO-Gruppe und
E eine Hydroxy- oder Ethoxygruppe darstellen,

(c) $Y_1$ eine -CO$CH_2$-Gruppe,
$Y_2$ eine 1,4-Phenylengruppe,
$Y_3$ eine -O$CH_2$CO-Gruppe und
E eine Hydroxy- oder tert.Butyloxygruppe darstellen,

$R_a$ eine 4-Pyridylgruppe,

$Y_1$ eine -CO-, -CO-CO-, -$A_1$-CO-, -CO-$A_1$- oder -CO-$A_1$-CO-Gruppe, in denen

$A_1$ eine gegebenenfalls durch eine $C_{1-5}$-Alkyl-, Phenyl- oder Phenyl-$C_{1-2}$-alkylgruppe substituierte n-$C_{1-5}$-Alkylengruppe darstellt, wobei die Phenylkerne der vorstehend erwähnten Phenyl- und Phenylalkylgruppen jeweils zusätzlich durch eine Hydroxy- oder Methoxygruppe substituiert sein können,

$Y_2$ eine 1,4-Phenylen-, 4-Piperidinylen-, 1,4-Piperazinylen- oder -$NR_1$-B-Gruppe, wobei die Verknüpfung mit der $Y_1$-Gruppe über das Stickstoffatom der -$NR_1$-Gruppe erfolgt und

$R_1$ ein Wasserstoffatom, eine $C_{1-5}$-Alkyl-, Phenyl- oder Phenyl-$C_{1-2}$-alkylgruppe und

B eine 1,3-Phenylen-, 1,4-Phenylen-, 1,3-Cyclohexylen-, 1,4-Cyclohexylen-, 3-Piperidinylen- oder 4-Piperidinylengruppe darstellen,

$Y_3$ eine -CO-, -$A_2$-CO-, -$NR_2$-$A_3$-CO- oder -O-$A_3$-CO-Gruppe, in denen

$A_2$ eine gegebenenfalls durch eine $C_{1-3}$-Alkyl-, Phenyl- oder Phenyl-$C_{1-2}$-alkylgruppe substituierte n-$C_{1-4}$-Alkylengruppe,

$A_3$ eine gegebenenfalls durch eine $C_{1-5}$-Alkyl-, Phenyl- oder Phenyl-$C_{1-3}$-alkylgruppe substituierte n-$C_{1-3}$-Alkylengruppe und

$R_2$ ein Wasserstoffatom, eine $C_{1-3}$-Alkyl-, $C_{1-5}$-Alkoxycarbonyl- oder $C_{1-3}$-Alkanoylgruppe darstellen sowie die Verknüpfung der -$A_2$-CO-Gruppe über den Rest $A_2$, der -$NR_2$-$A_3$-CO-Gruppe über die -$NR_2$-Gruppe und der -O-$A_3$-CO-Gruppe über das Sauerstoffatom mit dem Rest $Y_2$ erfolgt, wobei jedoch eine -$NR_2$- oder -O-$A_3$-CO-Gruppe nicht mit einem Stickstoffatom des Restes $Y_2$ verknüpft sein kann,

und E eine Hydroxygruppe, eine Alkoxygruppe mit 1 bis 5 Kohlenstoffatomen, eine Cycloalkoxygruppe mit 5 bis 7 Kohlenstoffatomen oder eine $R_5$-CO-O-($R_3CR_4$)-O-Gruppe, in der

$R_3$ ein Wasserstoffatom, eine $C_{1-3}$-Alkyl- oder $C_{5-7}$-Cycloalkylgruppe,

$R_4$ ein Wasserstoffatom und

$R_5$ eine $C_{1-5}$-Alkyl- oder $C_{1-3}$-Alkoxygruppe darstellen,

oder E eine α-Aminogruppe einer natürlichen Aminosäure oder deren Estern mit einem $C_{1-6}$-Alkanol oder Benzylalkohol

bedeuten,
deren Tautomere, deren Stereoisomere, einschließlich ihrer Gemische, und deren Salze.

Ganz besonders bevorzugte Verbindungen der obigen allgemeinen Formel I sind diejenigen, in denen mit der Maßgabe, daß nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO-$-Gruppe,
$Y_2$ eine 1,3- oder 1,4-Phenylengruppe,
$Y_3$ eine-$CH_2CO-$, $-CH_2CH_2CO-$ oder $-OCH_2CO-$-Gruppe und
E eine Hydroxy-, Methoxy- oder Ethoxygruppe oder

(b) $Y_1$ eine $-CH_2CO-$-Gruppe,
$Y_2$ eine 4-Piperidinylengruppe,
$Y_3$ eine -CO-, $-CH_2CO-$ oder $-OCH_2CO-$-Gruppe und
E eine Hydroxy- oder Ethoxygruppe darstellen,

(c) $Y_1$ eine $-COCH_2-$Gruppe,
$Y_2$ eine 1,4-Phenylengruppe,
$Y_3$ eine $-OCH_2CO-$-Gruppe und
E eine Hydroxy- oder tert.Butyloxygruppe darstellen,

$R_a$ eine 4-Pyridylgruppe,
$Y_1$ eine -CO-, -COCO-, $-A_1-CO-$, $-CO-A_1-$ oder $-CO-CH_2-CO-$-Gruppe, in denen
$A_1$ eine gegebenenfalls durch eine Methyl- oder Methoxyphenylgruppe substituierte n-$C_{1-4}$-Alkylengruppe darstellt,
$Y_2$ eine 1,4-Phenylen-, 4-Piperidinylen-, 1,4-Piperazinylen- oder $-NR_1-B-$Gruppe, wobei die Verknüpfung mit der $Y_1$-Gruppe über das Stickstoffatom der $-NR_1-$Gruppe erfolgt und
$R_1$ ein Wasserstoffatom und
B eine 1,3-Phenylen-, 1,4-Phenylen-, 1,3-Cyclohexylen-, 1,4-Cyclohexylen- oder 4-Piperidinylengruppe darstellen,
$Y_3$ eine -CO-, $-A_2-CO-$, $-NR_2-A_3-CO-$ oder $-O-A_3-CO-$-Gruppe, in denen
$A_2$ eine n-$C_{1-3}$-Alkylengruppe,
$A_3$ eine $C_{1-2}$-Alkylengruppe und
$R_2$ ein Wasserstoffatom, eine Methyl-, Benzyl-, Phenylethyl- oder Acetylgruppe darstellen sowie die Verknüpfung der $-A_2-CO-$Gruppe über den Rest $A_2$, der $-NR_2-A_3-CO-$Gruppe über die $-NR_2-$Gruppe und der $-O-A_3-CO-$Gruppe über das Sauerstoffatom mit dem Rest $Y_2$ erfolgt, wobei jedoch eine $-NR_2-$ oder $-O-A_3-CO-$Gruppe nicht mit einem Stickstoffatom des Restes $Y_2$ verknüpft sein kann,
und E eine Hydroxygruppe, eine Alkoxygruppe mit 1 bis 4 Kohlenstoffatomen, eine Cycloalkoxygruppe mit 5 bis 7 Kohlenstoffatomen oder eine $R_5-CO-O-(R_3CR_4)-O-$Gruppe, in der
$R_3$ ein Wasserstoffatom oder eine $C_{1-3}$-Alkylgruppe,
$R_4$ ein Wasserstoffatom und
$R_5$ eine $C_{1-5}$-Alkyl- oder $C_{1-3}$-Alkoxygruppe darstellen,
oder E eine Glycinylgruppe oder deren Methylester bedeuten,
deren Tautomere, deren Stereoisomere, einschließlich ihrer Gemische, und deren Salze.

Als besonders wertvolle Verbindungen seien beispielsweise folgende erwähnt:

(a) [4-trans-[3-[4-(4-Pyridyl)-piperazin-1-yl]propionyl]amino]cyclohexancarbonsäure,

(b) 3-[4-trans-[4-(4-Pyridyl)-piperazin-1-yl]carbonylamino]cyclohexylpropionsäure,

(c) 3-[4-trans-[4-(4-Pyridyl)-piperazin-1-yl]malonylamino]cyclohexylcarbonsäure,

(d) 3-[4-[4-(4-Pyridyl)-piperazin-1-yl]carbonylamino]piperidinopropionsäure,

(e) [4-[4-(4-Pyridyl)-piperazin-1-yl]carbonylamino]piperidinoessigsäure,

(f) [4-trans-[3-[4-(4-Pyridyl)-piperazin-1-yl]propionyl]amino]cyclohexancarbonsäure-methylester,

(g) 3-[4-trans-[4-(4-Pyridyl)-piperazin-1-yl]carbonylamino]cyclohexylpropionsäure-methylester,

(h) [4-trans-[4-(4-Pyridyl)-piperazin-1-yl]malonylamino]cyclohexylcarbonsäure-methylester,

(i) 4-[[4-(4-Pyridyl)-piperazin-1-yl]carbonylamino]piperidinoessigsäure-methylester,

(j) [4-trans-[[4-(4-Pyridyl)-piperazin-1-yl]acetyl]amino]cyclohexancarbonsäurecyclohexylester,

(k) [4-trans-[[4-(4-Pyridyl)-piperazin-1-yl]acetyl]amino]cyclohexancarbonsäure-isobutylester,

deren Tautomere und deren Salze.

Erfindungsgemäß erhält man beispielsweise die neuen Verbindungen nach folgenden Verfahren:

a. Umsetzung einer Verbindung der allgemeinen Formel

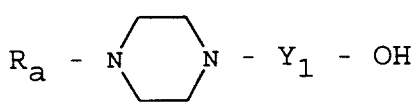

, (II)

in der
$R_a$ und $Y_1$ wie eingangs definiert sind, oder deren reaktionsfähigen Derivaten mit einer Verbindung der allgemeinen Formel

$$H - Y_2' - Y_3 - E' \quad ,(III)$$

in der
$Y_3$ wie eingangs definiert ist,
$Y_2'$ mit Ausnahme der Phenylengruppe die für $Y_2$ eingangs erwähnten Bedeutungen aufweist und
E' eine $C_{1-6}$-Alkoxy-, Phenyl-$C_{1-3}$-alkoxy- oder $C_{5-7}$-Cycloalkoxygruppe darstellt.

Die Umsetzung einer Carbonsäure der allgemeinen Formel II, in der $Y_1$ eine -$A_1$-CO- oder -CO-$A_1$-CO-Gruppe darstellt, wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder in einem entsprechenden Amin der allgemeinen Formel III gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxybenztriazol, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechenden reaktionsfähigen Verbindung der allgemeinen Formel II wie deren Ester, Imidazolide oder Halogeniden mit einem Amin der allgemeinen Formel III wird vorzugsweise in einem entsprechenden Amin als Lösungsmittel gegebenenfalls in Gegenwart eines weiteren Lösungsmittels wie Methylenchlorid oder Ether und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

b. Zur Herstellung einer Verbindung der allgemeinen Formel I, in der mindestens einer der Reste $R_2$ oder E ein reaktionsfähiges Wasserstoffatom mit der Maßgabe enthalten muß, daß E mit Ausnahme der $R_5$-CO-O-($R_3CR_4$)-O-Gruppe die für E eingangs erwähnten Bedeutungen aufweist:
Überführung einer Verbindung der allgemeinen Formel

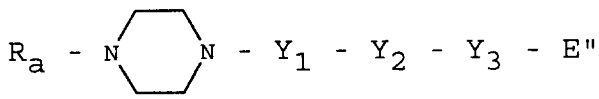 , (IV)

in der

$R_a$ und $Y_1$ bis $Y_3$ wie eingangs definiert sind und

E" eine Hydroxygruppe oder zusammen mit der benachbarten Carbonylgruppe des Restes $Y_3$ eine mittels Hydrolyse, Behandeln mit einer Säure oder Base, Thermolyse oder Hydrogenolyse abspaltbaren Schutzrest in eine Carboxylgruppe überführbare Gruppe bedeutet, wobei jedoch mindestens einer der Reste $NR_2$ oder E" einen abspaltbaren Rest enthalten muß,

in eine Verbindung der allgemeinen Formel I, in der mindestens einer der Reste $R_2$ oder E ein reaktionsfähiges Wasserstoffatom mit der Maßgabe enthalten muß, daß E mit Ausnahme der $R_5$-CO-O-($R_3CR_4$)-O-Gruppe die für E eingangs erwähnten Bedeutungen aufweist.

Als Schutzgruppen für eine Hydroxygruppe einer Carboxygruppe können beispielsweise die funktionelle Derivate einer Carboxygruppe wie deren unsubstituierte oder substituierte Amide, Ester, Thioester, Trimethylsilylester, Orthoester oder Iminoester mittels Hydrolyse in eine Carboxylgruppe, Ester mit tertiären Alkoholen, z.B. der tert. Butylester, mittels Behandlung mit einer Säure oder Thermolyse in eine Carboxylgruppe und

Ester mit Aralkanolen, z.B. der Benzylester, mittels Hydrogenolyse in eine Carboxylgruppe übergeführt werden.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Unter den vorstehend erwähnten Reaktionsbedingungen können gegebenenfalls vorhandene N-Acylamino- oder $C_{1-5}$-Alkoxycarbonylgruppen wie eine N-Trifluoracetylamino- oder tert.Butyloxycarbonylgruppe in die entsprechenden Aminogruppen übergeführt werden.

Bedeutet E" in einer Verbindung der Formel IV beispielsweise die tert. Butyloxycarbonylgruppe, so kann die tert.Butylgruppe auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden. Bei den vorstehend erwähnten Reaktionsbedingungen können gegebenenfalls vorhandene N-tert.Butyloxycarbonylaminogruppen in die entsprechenden Aminogruppen übergeführt werden.

Bedeutet E" in einer Verbindung der Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe in eine Aminogruppe, eine Benzyloxygruppe in eine Hydroxygruppe und eine N-Benzylamino-, N-Benzylimino-, N-Benzyloxycarbonylamino- oder N-Benzyloxycarbonyliminogruppe in eine entsprechende Amino- oder Iminogruppe übergeführt werden.

c. Zur Herstellung einer Verbindung der allgemeinen Formel I, in der $Y_2$ mit Ausnahme der Phenylengruppe wie eingangs erwähnt definiert ist und $Y_3$ eine -$A_2$-CO- Gruppe, in der $A_2$ eine gegebenenfalls durch eine $C_{1-5}$-Alkyl-, Phenyl- oder Phenyl-$C_{1-3}$-alkylgruppe substituierte n-$C_{2-4}$-Alkenylengruppe bedeutet, darstellen:
Umsetzung einer Verbindung der allgemeinen Formel

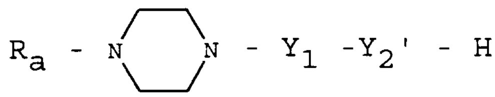 , (V)

in der
$R_a$ und $Y_1$ wie eingangs definiert sind und
$Y_2$' mit Ausnahme der Phenylen-, 3-Piperidinylen- oder 4-Piperidinylengruppe die für $Y_2$ eingangs erwähnten Bedeutungen aufweist, mit einer Verbindung der allgemeinen Formel

$$A_2' - CO - E \quad ,(VI)$$

in der
E wie eingangs definiert ist und
$A_2$' eine gegebenenfalls durch eine $C_{1-5}$-Alkyl-, Phenyl- oder Phenyl-$C_{1-3}$-alkylgruppe substituierte n-$C_{2-4}$-Alkenylengruppe darstellt.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer tertiären organischen Base wie N-Ethyl-diisopropylamin oder N-Methyl-morpholin bei Temperaturen zwischen -30 und 150°C, vorzugsweise jedoch bei Temperaturen zwischen 0 und 100°C, durchgeführt.

d. Umsetzung einer Verbindung der allgemeinen Formel

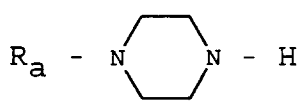 , (VII)

in der
Ra wie eingangs definiert ist, mit einer Verbindung der allgemeinen Formel

$$Z_1 - Y_1 - Y_2 - Y_3 - E \quad ,(VIII)$$

in der
$Y_1$, $Y_2$, $Y_3$ und E wie eingangs definiert sind und
$Z_1$ eine nukleophile Austrittsgruppe wie ein Halogenatom, eine Hydroxy- oder Sulfonsäureestergruppe, z.B. ein Chlor-, Brom- oder Jodatom, eine Hydroxy-, Imidazolyl-, 4-Nitrophenyloxy-, Methansulfonyloxy- oder p-Toluolsulfonyloxygruppe, oder auch, wenn $Y_1$ eine Carbonylgruppe darstellt,
$Z_1$ zusammen mit $R_1$ eine weitere Kohlenstoff-Stickstoff-Bindung bedeutet.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base oder gegebenfalls in Gegenwart eins wasserentziehendes Mittels bei Temperaturen zwischen -30 und 200°C durchgeführt.

Mit einer Verbindung der allgemeinen Formel VIII, in der $Z_1$ eine nukleophile Austrittsgruppe darstellt, oder mit einem Isocyanat der allgemeinen Formel VIII wird die Umsetzung vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumcarbonat, Kalium-tert.butylat oder N-Ethyldiisopropylamin oder gegebenenfalls in Gegenwart eines wasserentziehenden Mittels wie Triphenylphosphin/Azodicarbonsäurediethylester bei Temperaturen zwischen -20 und 100°C, vorzugsweise bei Temperaturen zwischen 0 und 60°C, durchgeführt. e. Zur Herstellung einer Verbindung der allgemeinen Formel I,

in der E eine $C_{1-6}$-Alkoxy-, Phenyl-$C_{1-3}$-alkoxy-, $C_{5-7}$-Cycloalkoxy- oder $R_5$-CO-O-($R_3CR_4$)-O-Gruppe darstellt: Umsetzung einer Verbindung der allgemeinen Formel

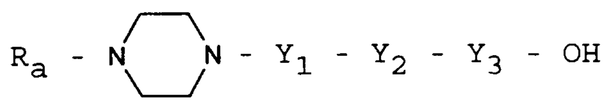

, (IX)

in der
$R_a$ und $Y_1$ bis $Y_3$ wie eingangs definiert sind, mit einer Verbindung der allgemeinen Formel

$$HO - R_b \qquad ,(X)$$

oder mit einer Verbindung der allgemein Formel

$$Z_2 - R_c \qquad ;(XI)$$

in denen
$R_b$ eine $C_{1-6}$-Alkyl-, Phenyl-$C_{1-3}$-alkyl- oder $C_{5-7}$-Cycloalkylgruppe,
$R_c$ eine eine $C_{1-6}$-Alkyl-, Phenyl-$C_{1-3}$-alkyl-, $C_{5-7}$-Cycloalkyl- oder $R_5$-CO-O-($R_3CR_4$)-Gruppe, in der
$R_3$, $R_4$ und $R_5$ wie eingangs definiert sind und
$Z_2$ eine Austrittsgruppe wie ein Halogenatom, z. B. ein Chlor- oder Bromatom, darstellen.

Mit einem Alkohol der allgemeinen Formel X wird die Umsetzung zweckmäßigerweise in einem Lösungsmittel oder Lösungsmittelgemisch wie Methylenchlorid, Benzol, Toluol, Chlorbenzol, Tetrahydrofuran, Benzol/Tetrahydrofuran oder Dioxan, vorzugsweise jedoch in einem Alkohol der allgemeinen Formel X, gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol- oder N,N'-Thionyldiimidazol oder Triphenylphosphin/Tetrachlorkohlenstoff, gegebenenfalls in Gegenwart einer Base wie Kaliumcarbonat, N-Ethyl-diisopropylamin oder N,N-Dimethylamino-pyridin zweckmäßigerweise bei Temperaturen zwischen 0 und 150°C, vorzugsweise bei Temperaturen zwischen 0 und 80°C, durchgeführt.

Mit einer Verbindung der allgemeinen Formel XI wird die Umsetzung zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyldiisopropylamin oder N-Methyl-morpholin, welche gleichzeitig auch als Lösungsmittel dienen können, oder gegebenenfalls in Gegenwart von Sllberkarbonat oder Silberoxid bei Temperaturen zwischen -30 und 100°C, vorzugsweise jedoch bei Temperaturen zwischen -10 und 80°C, durchgeführt.

f. Umsetzung einer Verbindung der allgemeinen Formel

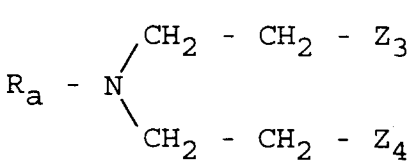

, (XII)

in der
$R_a$ wie eingangs definiert ist,
$Z_3$ und $Z_4$, die gleich oder verschieden sein können, nukleophile Austrittsgruppen wie Halogenatome oder Sulfonyloxygruppen, z.B. Chlor- oder Bromatome, Methansulfonyloxy- oder p-Toluolsulfonyloxygruppen, bedeuten, mit einer Verbindung der allgemeinen Formel

$$NH_2 - Y_1 - Y_2 - Y_3 - E \qquad ,(XIII)$$

in der
E, $Y_2$ und $Y_3$ wie eingangs definiert sind und
$Y_1$ eine $-A_1$-CO- oder $-A_2-SO_2$-gruppe darstellt, wobei $A_1$ und $A_2$ wie eingangs definiert sind.

Die Umsetzung wird zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino- oder Iminogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyl- oder Tetrahydropyranylgruppe und
als Schutzrest für eine Amino- oder Iminogruppe die Formyl-, Acetyl-, Trifluoracetyl-, Allyloxycarbonyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge oder wäßriger Lithiumhydroxid-Lösung gegebenenfalls in Gegenwart eines Lösungsmittels wie Tetrahydrofuran oder Methanol bei Temperaturen zwischen 0 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Allylgruppenakzeptors wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wäßrigem Ethanol und gegebenenfalls in Gegenwart einer Base wie 1,4-Diazabicyclo[2.2.2]octan bei Temperaturen zwischen 20 und 70°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalisch chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen sind teilweise literaturbekannt oder man erhält diese nach literaturbekannten Verfahren (siehe Beispiele I bis XXXI).

Wie bereits eingangs erwähnt, weisen die neuen Piperazinderivate der allgemeinen Formel I und deren Salze, insbesondere deren physiologisch verträglichen Salze mit anorganischen oder organischen Säuren oder Basen, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. Hemmung der Bindung von $^{3}$H-BIBU 52 an Humanthrombozyten:

Eine Suspension von Humanthrombozyten in Plasma wird mit $^{3}$H-BIBU 52 [= (3S,5S)-5-[(4'-Amidino-4-biphenylyl)oxymethyl]3-[(carboxyl)methyl]-2-pyrrolidinon[3-$^{3}$H-4-biphenylyl]], das den literaturbekannten Liganden $^{125}$J-Fibrinogen ersetzt, (siehe DE-A-4,214,245) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^{3}$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^{3}$H-BIBU 52 (Endkonzentration: 5 nM) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene Restplasma wird aus dem $^{14}$C-Gehalt bestimmt, der gebundene Ligand aus der $^{3}$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung:

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 5 | 0.21 | 0.26 |
| 5(6) | 0.25 | 0.16 |
| 5(7) | 0.23 | 0.25 |
| 5(8) | 0.41 | 0.60 |
| 5(9) | 0.43 | 4.40 |
| 1 | >100 | 3.32 |
| 4 | 0.55 | 0.39 |
| 1(6) | 0.38 | 3.60 |
| 4(1) | 48 | >10 |

Die neuen Verbindungen sind gut verträglich, da beispielsweise nach intravenöser Gabe von 30 mg/kg der erfindungsgemäßen Verbindungen der allgemeinen Formel I an der Maus keine toxischen Nebenwirkungen beobachtet werden konnten.

Auf Grund ihrer Hemmwirkung auf Zell-Zell- bzw. Zell-Matrix-Wechselwirkungen eignen sich die neuen Piperazinderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

3-[4-(4-Pyridyl)-piperazin-1-yl]propionsäure

a) 3-[4-(4-Pyridyl)-piperazin-1-yl]propionsäure-methylesterdihydrochlorid

Eine Lösung von 4 g (0,0245 Mol) 1-(4-Pyridyl)-piperazin, 21,1 g (0,245 Mol) Acrylsäure-methylester und 6,5 ml (0,0358 Mol) einer 40%igen methanolischen Lösung von Benzyltrimethylammoniumhydroxid in 50 ml Methanol und 50 ml Chloroform wird während 3 Stunden auf Rückfluß-Temperatur erhitzt. Anschließend wird das Lösungsmittel unter Wasserstrahlvakuum abdestilliert und der Rückstand zwischen Methylenchlorid und Wasser verteilt. Die Methylenchlorid-Extrakte werden über Natriumsulfat getrocknet und eingeengt. Der Rückstand wird in Ether gelöst und mit etherischer Salzsäure bis pH 3 angesäuert. Das ausgefallene Hydrochlorid wird abgesaugt, mit Ether gewaschen und unter Vakuum getrocknet.
Ausbeute: 6,4 g (81 % der Theorie),
Schmelzpunkt: >330°C
$R_f$-Wert: 0,45 (Kieselgel; Methylenchlorid/Methanol/konz. Ammoniak = 9:1:0,25)

b) 3-[4-(4-Pyridyl)-piperazin-1-yl]-propionsäure

Zu einer Lösung von 6 g (0,02 Mol) 3-[4(4-Pyridyl)piperazin-1-yl]propionsäure-methylester-dihydrochlorid in 80 ml Tetrahydrofuran und 100 ml Wasser gibt man eine Lösung von 4,2 g (0,1 Mol) Lithiumhydroxid in 100 ml Wasser und rührt zwei Stunden bei Raumtemperatur. Anschließend wird unter Vakuum zur Trockne eingeengt und der verbleibende Rückstand in absolutem Ethanol aufgenommen. Man versetzt mit 5,3 g (0,1 Mol) Ammonchlorid, saugt die ausgeschiedenen anorganischen Salze ab und engt die verbleibende Lösung zur Trockne ein. Der Rückstand wird mit Ether verrieben und abgesaugt.
Ausbeute: 3,4 g (72,8 % der Theorie),
Schmelzpunkt: ab 215°C
$R_f$-Wert: 0,21 (Kieselgel; Methylenchlorid/Methanol = 9:1)

Beispiel II

4-[4-(4-Pyridyl)-piperazin-1-yl]buttersäure-dihydrochlorid

a) 4-[4-(4-Pyridyl)-piperazin-1-yl]buttersäure-ethylesterdihydrochlorid

Zu einer Lösung von 6,4 g (0,039 Mol) 1-(4-Pyridyl)-piperazin in 150 ml Methanol gibt man unter Rühren 9,1 g (0,047 Mol) 4-Brombuttersäure-ethylester (6,7 ml) und 6,0 g (0,047 Mol) N-Ethyl-diisopropylamin (4,3 ml) und erhitzt die so entstandene Lösung während 48 Stunden auf Rückfluß-Temperatur. Anschließend wird die Lösung unter Vakuum eingeengt und der Rückstand zwischen Methylenchlorid und Wasser verteilt. Die organische Phase wird über Natriumsulfat getrocknet und unter Vakuum zur Trockne eingeengt. Der verbleibende Rückstand wird in Ethanol gelöst und mit etherischer Salzsäure bis pH 3 angesäuert. Man engt erneut unter Vakuum zur Trockne ein, verreibt den Rückstand mit Aceton und saugt die Kristalle ab.
Ausbeute: 6 g (43,7 % der Theorie).
Massenspektrum: $M^+ = 277$
$R_f$-Wert: 0,60 (Kieselgel; Methylenchlorid/Methanol/konz. Ammoniak = 9:1:0,25)

b) 4-[4-(4-Pyridyl)-piperazin-1-yl]buttersäure

6 g (0,017 Mol) 4-[4-(4-Pyridyl)-piperazin-1-yl]buttersäureethylester-dihydrochlorid werden in 120 ml halbkonzentrierter Salzsäure gelöst. Die Lösung wird während 18 Stunden bei Raumtemperatur stehen gelassen und anschließend unter Vakuum zur Trockne eingeengt. Der verbleibende Rückstand wird mit Aceton verrieben und der amorphe Festkörper abgesaugt und getrocknet.
Ausbeute: 5,2 g (94,2 % der Theorie),
Massenspektrum: $M^+ = 249$
$R_f$-Wert: 0,11 (Kieselgel; Methylenchlorid/Methanol/konz. Ammoniak = 4:1:0,2)

Beispiel III

[4-(4-Pyridyl)-piperazin-1-yl]essigsäure-dihydrochlorid

a) [(4-(4-Pyridyl)-piperazin-1-yl]essigsäure-methylesterhydrochlorid

Hergestellt aus 1-(4-Pyridyl)-piperazin und Bromessigsäuremethylester analog Beispiel IIa.

b) [4-(4-Pyridyl)-piperazin-1-yl]essigsäure-dihydrochlorid Hergestellt aus [4-(4-Pyridyl)-piperazin-1-yl]essigsäure-methylester-dihydrochlorid und halbkonzentrierter Salzsäure analog Beispiel IIb.

Beispiel IV

5-[4-(4-Pyridyl)-piperazin-1-yl]valeriansäure-dihydrochlorid

a) 5-[4-(4-Pyridyl)-piperazin-1-yl]valeriansäure-ethylester

Zu einer Lösung von 6,4 g (0,039 Mol) 1-(4-Pyridyl)-piperazin in 150 ml Ethanol gibt man unter Rühren 11,5 g (0,055 Mol) 5-Bromvaleriansäure-ethylester (8,7 ml) und 7,1 g (0,055 Mol) N-Ethyl-diisopropylamin (5,0 ml) und erhitzt die so entstandene Lösung während 48 Stunden auf Rückfluß-Temperatur. Anschließend wird die Lösung unter Vakuum eingeengt und der Rückstand zwischen Methylenchlorid und Wasser verteilt. Die organische Phase wird über Natriumsulfat getrocknet und unter Vakuum zur Trockne eingeengt.
Ausbeute: 10 g (87,5 % der Theorie), Öl
$R_f$-Wert: 0,60 (Kieselgel; Methylenchlorid/Methanol/konz. Ammoniak = 4:1:0,2)

b) 5-[4-(4-Pyridyl)-piperazin-1-yl]valeriansäure-dihydrochlorid

10 g (0,034 Mol) 5-[4-(4-Pyridyl)-piperazin-1-yl]valeriansäureethylester werden in 150 ml halbkonzentrierter Salzsäure gelöst. Diese Lösung wird während 18 Stunden bei Raumtemperatur stehen gelassen und anschließend unter Vakuum zur Trockne eingeengt. Man versetzt zweimal mit Aceton und engt jedesmal unter Vakuum zur Trockne ein, wobei man einen farblosen Schaum erhält, der als Rohprodukt weiter umgesetzt wird.
Ausbeute: 9,0 g (78 % der Theorie),
$R_f$-Wert: 0,09 (Kieselgel; Methylenchlorid/Methanol = 4:1)

Beispiel V

[4-(4-Pyridyl)-piperazin-1-yl]malonsäure

a) [4-(4-Pyridyl)-piperazin-1-yl]malonsäure-ethylester

Eine Lösung von 4,0 g (0,025 Mol) 1-(4-Pyridyl)-piperazin, 4,4 g (0,029 Mol) Malonsäure-ethylesterchlorid (3,8 ml) und 3,9 g (0,029 Mol) N-Ethyl-diisopropylamin (5 ml) in 150 ml trockenem Tetrahydrofuran wird während 6 Stunden auf Rückfluß-Temperatur erhitzt. Anschließend wird die Lösung unter Vakuum zur Trockene eingeengt und der Rückstand über eine Kieselgel-Säule gereinigt, wobei Methylenchlorid/Methanol/konz. Ammoniak = 9:0,5:0,05 und 9:1:0,1 als Elutionsmittel verwendet wird.
Ausbeute: 5 g (73,6% der Theorie),
Massenspektrum; $M^+$ = 277
$R_f$-Wert: 0,45 (Kieselgel; Methylenchlorid/Methanol/konz. Ammoniak = 9:1:0,1)

b) [4-(4-Pyridyl)-piperazin-1-yl]malonsäure

Eine Lösung von 5,0 g (0,018 Mol) [4-(4-Pyridyl)-piperazin-1-yl]malonsäure-ethylester in 100 ml halbkonzentrierter Salzsäure wird über Nacht bei Raumtemperatur stehen gelassen und anschließend unter Vakuum zur Trockne eingeengt. Der Rückstand wird dreimal mit Aceton versetzt und jeweils unter Vakuum zur Trockne eingeengt. Ausbeute: 4,7 g (89,5 % der Theorie),
Farbloser Schaum
Massenspektrum; $M^+$ = 249
$R_f$-Wert: 0,10 (Kieselgel; Methylenchlorid/Methanol/konz. Ammoniak = 4:1:0,2)

Beispiel VI

4-[[4-(4-Pyridyl)-piperazin-1-yl]carbonylamino]piperidin

a) 4-[[4-(4-Pyridyl)-piperazin-1-yl]carbonylamino]-N-benzylpiperidin

Zu einer Lösung von 8,2 g (0,05 Mol) N,N'-Carbonyldiimidazol und 4,3 g (0,063 Mol) Imidazol in 300 ml trockenem Dimethylformamid tropft man bei -5°C und unter Rühren eine Lösung von 8 g (0,042 Mol) 4-Amino-N-benzyl-piperidin (8,6 ml) in 20 ml trockenem Dimethylformamid und rührt eine weitere Stunde bei - 5°C und dann während einer Stunde bei Raumtemperatur. Anschließend tropft man 6,9 g (0,042 Mol) 4-Pyridyl-piperazin, gelöst in 50 ml Dimethylformamid, zu und läßt über Nacht bei Raumtemperatur rühren. Die Lösung wird unter Vakuum zur Trockne eingeengt und der Rückstand zwischen Essigester und Wasser verteilt. Die vereinigten organischen Extrakte werden getrocknet, eingeengt und über eine Kieselgel-Säule gereinigt, wobei Methylenchlorid/Methanol/konz. Ammoniak = 9:0,5:0,05 als Elutionsmittel verwendet wird.
Ausbeute: 5,0 g (31,2 % der Theorie),
Massenspektrum; $M^+$ = 379
$R_f$-Wert: 0,45 (Kieselgel; Methylenchlorid/Methanol/konz. Ammoniak = 9:1:0,1)

b) 4-[[4-(4-Pyridyl)-piperazin-1-yl]carbonylamino]piperidin

Eine Lösung von 5 g (0,13 Mol) 4-[[4-(4-Pyridyl)-piperazin-1-yl]carbonylamino]-N-benzyl-piperidin in 100 ml Methanol wird bei Raumtemperatur und unter einem Druck von 50 psi über Palladiumdihydroxid auf Kohle als Katalysator erschöpfend hydriert. Nach Abfiltrieren des Katalysators wird die verbleibende Lösung unter Vakuum zur Trockne eingeengt.
Ausbeute: 2,0 g (54 % der Theorie), Öl
Massenspektrum: $M^+$ = 289
$R_f$-Wert: 0,10 (Kieselgel; Methylenchlorid/Methanol/konz. Ammoniak = 9:1:0,1)

Beispiel VII

4-Amino-piperidin-1-yl-essigsäure-methylester-dihydrochlorid

a) 4-tert.Butyloxycarbonylamino-N-benzyl-piperidin

Zu einer Lösung von 50 g (0,26 Mol) 4-Amino-1-benzyl-piperidin in 300 ml trockenem Dioxan tropft man unter Rühren und Kühlen mit Wasser eine Lösung von 60 g (0,276 Mol) Di-tert.Butyldicarbonat in 150 ml trockenem Dioxan. Man rührt nach beendeter Zugabe 4 Stunden bei Raumtemperatur und engt unter Vakuum zur Trockne ein. Der verbleibende Rückstand wird mit wenig Ether und Petrolether verrieben, abgesaugt und mit Petrolether gewaschen.
Ausbeute: 70,6 g (92,6 % der Theorie),
Schmelzpunkt: 114-115°C
$R_f$-Wert: 0,60 (Kieselgel; Methylenchlorid/Methanol = 9:1)

b) 4-tert.Butyloxycarbonylamino-piperidin

Eine Lösung von 5 g (0,017 Mol) 4-tert.Butyloxycarbonylamino-N-benzyl-piperidin in 50 ml Methanol wird mit etherischer Salzsäure bis pH 6 angesäuert und über Palladium auf Kohle (10%ig) unter einem Wasserstoffdruck von 50 psi bei Raumtemperatur erschöpfend hydriert. Der Katalysator wird abfiltriert, das Filtrat unter Vakuum zur Trockne eingeengt, der Rückstand mit Ether verrieben und der Festkörper abgesaugt.
Ausbeute: 3,3 g (95,7 % der Theorie),
Massenspektrum: $M^+$ = 200
$R_f$-Wert: 0,13(Kieselgel; Methylenchlorid/Methanol = 9:1)

c) 4-tert.Butyloxycarbonylamino-piperidin-1-yl-essigsäuremethylester

Eine Lösung von 3,0 g (0,013 Mol) 4-tert.Butyloxycarbonylamino-piperidin, 1,9 g (0,13 Mol) Bromessigsäure-methylester (1,2 ml) und 2,6 g (0,025 Mol) Triethylamin (3,4 ml) wird über Nacht bei Raumtemperatur gerührt. Anschließend wird unter Vakuum zur Trockne eingeengt und der Rückstand zwischen Essigester und Wasser verteilt. Die organische Phase wird über Natriumsulfat getrocknet und eingeengt.
Ausbeute: 3,1 g (89,8 % der Theorie),

Massenspektrum: $M^+$ = 272
$R_f$-Wert: 0,43 (Kieselgel; Methylenchlorid/Methanol = 9:1)

d) 4-Aminopiperidin-1-yl-essigsäure-methylester-dihydrochlorid

Eine Lösung von 3,1 g (0,011 Mol) 4-tert.Butyloxycarbonylamino-piperidinessigsäure-methylester in 30 ml Methanol wird mit 30 ml etherischer Salzsäure angesäuert und über Nacht bei Raumtemperatur stehen gelassen. Anschließend engt man unter Vakuum zur Trockne ein, verreibt den Rückstand mit Ether und saugt den Festkörper ab.
Ausbeute: 2,4 g (100 % der Theorie),
Massenspektrum: $M^+$ = 140
$R_f$-Wert: 0,10 (Kieselgel; Methylenchlorid/Methanol = 9:1)

Beispiel VIII

N-[4-Nitrophenyloxycarbonyl]-3-(4-piperidinyl)-propionsäuremethylester

Zu einer Lösung von 4,75 g (0,0229 Mol) 3-(4-Piperidinyl)-propionsäure-methylester und 4,93 g (0,0229 Mol) Chlorameisensäure-p-nitrophenylester in 200 ml trockenem Tetrahydrofuran tropft man bei 0°C und unter Rühren 8 ml (0,0573 Mol) Triethylamin und läßt über Nacht bei Raumtemperatur rühren. Anschließend erhitzt man während 4 Stunden auf Raumtemperatur und engt unter Vakuum zur Trockne ein. Der Rückstand wird zwischen Methylenchlorid und Wasser verteilt, die organische Phase abgetrennt, getrocknet und eingeengt. Der verbleibende Rückstand wird über eine Kieselgel-Säule gereinigt, wobei Methylenchlorid als Elutionsmittel verwendet wird.
Ausbeute: 9 g Öl, das als Verunreinigung 4-Nitrophenol enthält.
Massenspektrum: $M^+$ = 336
$R_f$-Wert: 0,93 (Kieselgel; Methylenchlorid/Methanol = 9:1)

Beispiel IX

N-tert.Butyloxycarbonyl-N-[4-[[4-(4-pyridyl)-piperazin-1-yl]carbonyl]piperidinyl]-β-alanin-methylester

Zu einer Lösung von 5 g (0,0155 Mol) N-tert.Butyloxycarbonyl-N-(4-piperidinyl)-β-alanin-methylester-hydrochlorid und 4,8 ml (0,0341 Mol) Triethylamin in 150 ml trockenem Tetrahydrofuran tropft man bei Raumtemperatur und unter Rühren eine Lösung von 4,1 g (0,0186 Mol) Chlorameisensäure-p-nitrophenylester in 20 ml trockenem Tetrahydrofuran und läßt weitere 4 Stunden bei Raumtemperatur rühren. Das ausgefallene Triethylammoniumchlorid wird abgesaugt. Die verbleibende Lösung wird mit 2,53 g (0,0155 Mol) 1-(4-Pyridyl)-piperazin und 2,8 ml (0,0155 Mol) N-Ethyldiisopropylamin versetzt und über Nacht bei Raumtemperatur stehen gelassen. Nach Einengen zur Trockne wird der verbleibende ölige Rückstand während 6 Stunden auf 140°C erhitzt. Da laut Dünnschicht-Chromatogramm die Umsetzung noch nicht vollständig war, werden nochmals 1 g (0,0061 Mol) N-Ethyl-diisopropylamin zugegeben und weitere 6 Stunden auf 140°C erhitzt. Nach dem Abkühlen wird mit Petrolether verrieben, abdekantiert und der Rückstand zwischen Essigester und Wasser verteilt. Die Essigester-Phase wird mit 1N Natriumbicarbonat-Lösung gewaschen, getrocknet und eingeengt. Der Rückstand wird über eine Kieselgel-Säule gereinigt, wobei Methylenchlorid mit 2,5 % Methanol und Methylenchlorid mit 5 % Methanol und 0,4 % konz. Ammoniak als Elutionsmitel verwendet werden.
Ausbeute: 2,36 g (32 % der Theorie),
Massenspektrum: $(M+H)^+$ = 476
$R_f$-Wert: 0,40 (Kieselgel; Methylenchlorid/Methanol = 9:1)

Beispiel X

N-(4-Nitrophenyloxycarbonyl)-4-(4-piperidinyl)]-buttersäuremethylester

Hergestellt aus 4-(4-Piperidinyl)]-buttersäuremethylester-hydrochlorid, Chlorameisensäure-p-nitrophenylester und N-Ethyldiisopropylamin analog Beispiel VIII.
Öl, das langsam kristallisiert.
$R_f$-Wert: 0,11 (Kieselgel; Methylenchlorid/Methanol = 9:1)

Beispiel XI

N-tert.Butyloxycarbonyl-N-[[4-[4-(4-pyridyl)-piperazin-1-yl]carbonyl]piperidinyl]glycin-methylester

Hergestellt aus N-tert.Butyloxycarbonyl-N-[4-[1-(4-nitrophenyloxycarbonyl)-piperidinyl]]-glycinmethylester (Herstellung analog Beispiel VIII) und 1-(4-Pyridyl)-piperazin analog Beispiel IX.

Beispiel XII

N-tert.Butyloxycarbonyl-N-(4-piperidinyl)-β-alanin-methylesterhydrochlorid

a) N-[1-Benzyl-4-piperidinyl]-β-alanin-methylester-hydrochlorid

Eine Lösung von 50 g (0,263 Mol) 4-Amino-1-benzyl-piperidin und 28,5 ml (0,263 Mol) Acrylsäure-ethylester in 300 ml Methanol wird 4 Stunden lang auf Rückfluß-Temperatur erhitzt. Anschließend engt man unter Vakuum zur Trockne ein, löst den Rücksktand in Aceton, säuert mit etherischer Salzsäure bis pH 3 an und engt erneut unter Vakuum zur Trockne ein. Der verbleibende Rückstand wird mit Aceton verrieben. Das ausgeschiedene kristalline Produkt wird abgesaugt und getrocknet.
Ausbeute: 48,7 g (50,2 % der Theorie),
Schmelzpunkt: 172-180°C (Zers.)
$R_f$-Wert: 0,60 (Kieselgel; Methylenchlorid/Methanol = 9:1)

b) N-[1-Benzyl-4-piperidinyl]-N-tert.butyloxycarbonyl-β-alaninmethylester-hydrochlorid

Eine Lösung aus 25 g (0,0716 Mol) N-[1-Benzyl-4-piperidinyl]-β-alanin-methylester-hydrochlorid, 15,8 g (0,072 Mol) Di-tert.butyldicarbonat und 20 ml (0,138 Mol) Triethylamin in 100 ml Dioxan und 100 ml Wasser wird während 48 Stunden bei Raumtemperatur stehen gelassen. Anschließend wird unter Vakuum zur Trockne eingeengt und der Rückstand zwischen Essigester und Wasser verteilt. Die organische Phase wird über Natriumsulfat getrocknet und eingeengt. Der verbleibende Rückstand wird in Ethanol gelöst und mit etherischer Salzsäure bis pH 6 angesäuert. Man engt die Lösung unter Vakuum zur Trockne ein, verrührt den Rückstand mit Aceton und saugt den Festkörper ab.
Ausbeute: 24,1 g (81,5 % der Theorie),
Schmelzpunkt: 196-197°C (Zers.)
$R_f$-Wert: 0,80 (Kieselgel; Methylenchlorid/Methanol = 9:1)

c) N-tert.Butyloxycarbonyl-N-(4-piperidinyl)-β-alanin-methylester-hydrochlorid

24 g (0,05 Mol) N-[1-Benzyl-4-piperidinyl]-N-tert.butyloxycarbonyl-β-alanin-methylester-hydrochlorid werden in 900 ml Methanol bei Raumtemperatur und einem Wasserstoffdruck von 50 psi über Palladium auf Kohle (10%ig) als Katalysator erschöpfend hydriert. Der Katalysator wird abgesaugt und die Lösung unter Vakuum zur Trockne eingeengt.
Ausbeute: 20,4 g Öl,
$R_f$-Wert: 0,17 (Kieselgel; Methylenchlorid/Methanol = 9:1)

Beispiel XIII

N-tert.Butyloxycarbonyl-N-(4-piperidinyl)-glycin-methylesterhydrochlorid

a) N-[1-Benzyl-4-piperidinyl]-glycin-methylester-dihydrochlorid

Hergestellt aus 4-Amino-1-benzyl-piperidin, Bromessigsäuremethylester und N-Ethyl-diisopropylamin.

b) N-[1-Benzyl-4-piperidinyl]-N-tert.butyloxycarbonyl-glycinmethylester-dihydrochlorid

Hergestellt aus N-[1-Benzyl-4-piperidinyl]-glycin-methylesterhydrochlorid, Di-tert.butyldicarbonat und Triethylamin.

c) N-tert.Butyloxycarbonyl-N-(4-piperidinyl)-glycinmethylesterhydrochlorid

Hergestellt aus N-[1-Benzyl-4-piperidinyl]glycin-methylesterhydrochlorid durch erschöpfendes Hydrieren über Palladium auf Kohle (10%ig).

Beispiel XIV

N-Methyl-N-(4-piperidinyl)-β-alaninmethylester-dihydrochlorid

a) N-[1-Benzyl-4-piperidinyl]-N-methyl-β-alaninmethylester-dihydrochlorid

Eine Suspension von 28,8 g (0,026 Mol) N-[1-Benzyl-4-piperidinyl]-β-alaninmethylester-dihydrochlorid, 2,7 g (0,09 Mol) Paraformaldehyd und 5,2 g (0,083 Mol) Natriumcyanoborhydrid in 100 ml Ethanol wird während 24 Stunden bei Raumtemperatur gerührt. Anschließend verdünnt man mit Wasser und säuert mit 1n Salzsäure bis pH2 an. Man extrahiert mit Essigester, macht die wässrige Phase mit verdünnter Natronlauge alkalisch und extrahiert erschöpfend mit Methylenchlorid. Die vereinigten Methylenchlorid-Phasen werden getrocknet und unter Vakuum zur Trockne eingeengt. Der Rückstand wird über eine Kieselgel-Säule gereinigt, wobei Methylenchlorid mit 3 % und mit 5 % Methanol als Elutionsmittel verwendet wird. Die vereinigten Eluate werden mt etherischer Salzsäure auf pH3 angesäuert und unter Vakuum zur Trockne eingeengt. Der Rückstand wird mit Aceton versetzt und abgesaugt.
Ausbeute: 20,8 g (69,5 % der Theorie)
Schmelzpunkt: 224-227°C
$R_f$-Wert: 0,60 (Kieselgel; Methylenchlorid/Methanol = 4:1)

b) N-Methyl-N-(4-piperidinyl)-β-alaninmethylester-dihydrochlorid

Hergestellt durch Hydrieren von N-[1-Benzyl-4-piperidinyl]-N-methyl-β-alaninmethylester-dihydrochlorid mit Palladium auf Kohle (10%ig).
Ausbeute: 15,8 g (95,4 % der Theorie)
Schmelzpunkt: 194-195°C (Zers.)
$R_f$-Wert: 0,09 (Kieselgel; Methylenchlorid/Methanol = 9:1)
Analog Beispiel XIV können folgende Verbindungen hergestellt werden:

(1) N-Methyl-N-(4-piperidinyl)-glycinmethylester-dihydrochlorid

a) N-[1-Benzyl-4-piperidinyl]-N-methyl-glycinmethylester-dihydrochlorid
Hergestellt aus N-[1-Benzyl-4-piperidinyl]glycin-methylesterdihydrochlorid, Paraformaldehyd und Natriumcyanoborhydrid.

b) N-Methyl-N-(4-piperidinyl)-glycinmethylester-dihydrochlorid Hergestellt aus N-[1-Benzyl-4-piperidinyl]-N-methylglycinmethylester-dihydrochlorid durch erschöpfendes Hydrieren über Palladium auf Kohle (10%ig).

(2) N-(2-Phenylethyl)-N-(4-piperidinyl)-β-alanin-methylesterdihydrochlorid

a) N-[1-Benzyl-4-piperidinyl]-N-(2-phenylethyl)-β-alanin-methylester-dihydrochlorid
Hergestellt aus N-[1-Benzyl-4-piperidinyl]-β-alanin-methylester-dihydrochlorid, Phenylacetaldehyd und Natriumcyanoborhydrid.

b) N-(2-Phenylethyl)-N-4-(piperidinyl)-β-alanin-methylesterdihydrochlorid
Hergestellt aus N-[1-Benzyl-4-piperidinyl]-N-(2-phenylethyl)-β-alanin-methylester-dihydrochlorid durch erschöpfendes Hydrieren über Palladium auf Kohle (10%ig).

Beispiel XV

N-Acetyl-N-(4-piperidinyl)-β-alanin-methylester-hydrochlorid

a) N-Acetyl-N-[1-benzyl-4-piperidinyl]-β-alanin-methylesterhydrochlorid

Eine Lösung von 25 g (0,0716 Mol) N-(1-Benzyl-4-piperidinyl)-β-alanin-methylester-hydrochlorid, 20 ml (0,143 Mol) Triethylamin und 8,1 ml (0,0859 Mol) Acetanhydrid in 300 ml Methanol wird über Nacht bei Raumtemperatur stehen gelassen und anschließend unter Vakuum eingeengt. Der Rückstand wird in Wasser gelöst, mit 2n Natronlauge auf pH 8 eingestellt und mit Essigester erschöpfend extrahiert. Die vereinigten Essigester-Extrakte werden getrocknet und unter Vakuum zur Trockne eingeengt. Der Rückstand wird über eine Kieselgel-Säule mit Methylenchlorid, das 3 % Methanol enthält, gereinigt. Die Eluate werden eingeengt, der Rückstand in Aceton gelöst, mit etherischer Salzsäure auf pH 6 angesäuert und eingeengt. Der Rückstand wird mit Aceton/Ether zur Kristallisation gebracht.

Ausbeute: 19 g (74,7 % der Theorie),
Schmelzpunkt: 138-140°C (Zers.)
$R_f$-Wert: 0,50 (Kieselgel; Methylenchlorid/Methanol = 9:1)

b) N-Acetyl-N-(4-piperidinyl)-β-alanin-methylester-hydrochlorid

Hergestellt analog Beispiel XIIc durch Hydrieren mit Palladium auf Kohle (10%ig).
Ausbeute: 13,2 g (93,2 % der Theorie),
Sehr hygroskopischer Festkörper
Massenspektrum: $M^+$ = 228
$R_f$-Wert: 0,09 (Kieselgel; Methylenchlorid/Methanol = 9:1)

Beispiel XVI

4-(4-Piperidinyl)-buttersäuremethylester-hydrochlorid

a) 2-[2-(4-Pyridyl)-ethyl]malonsäue-diethylester-hydrochlorid

In 180 ml absolutem Ethanol löst man 13,4 g(0,583 Mol) Natrium und gibt zu der so entstandenen Lösung portionsweise 204 ml (1,35 Mol) Malonsäure-diethylester, wobei sich ein farbloser Niederschlag bildet. Diesen Niederschlag bringt man durch Erwärmen auf 30-40°C und Verdünnen mit absolutem Ethanol in Lösung und tropft innerhalb von 1,5 Stunden unter Rühren eine Lösung von 63 ml (0,583 Mol) 4-Vinylpyridin in 120 ml absolutem Ethanol zu. Nach beendeter Zugabe erhitzt man während 3 Stunden auf Rückflußtemperatur, engt anschließend auf ein kleines Volumen ein und verdünnt mit 450 ml halbkonzentrierter Salzsäure. Man extrahiert zweimal mit Ether, um überschüssigen Malonsäurediethylester zu entfernen, stellt die wäßrige Phase mit Natriumcarbonat alkalisch und extrahiert erschöpfend mit Methylenchlorid. Die vereinigten organischen Phasen werden getrocknet und eingeengt. Der Rückstand wird über eine Kieselgel-Säule gereinigt, wobei Essigester/Cyclohexan = 1:1 als Elutionsmittel verwendet wird. Der ölige Rückstand (78,6 g = 50,8 % der Theorie) wird in Aceton gelöst und mit etherischer Salzsäure bis pH 3,5 angesäuert und eingeengt. Der Rückstand kristallisiert über Nacht, wird mit Aceton/Ether verrieben und abgesaugt.
Ausbeute: 65 g (37 % der Theorie),
$R_f$-Wert: 0,80 (Kieselgel; Methylenchlorid/Methanol = 9:1)

b) 2-[2-(4-Piperidinyl)-ethyl]malonsäure-diethylester-hydrohlorid

64,5 g (0,21 Mol) 2-[2-(4-Pyridyl)-ethyl]malonsäure-diethylester-hydrochlorid werden in 400 ml absoluten Ethanol bei Raumtemperatur und einem Wasserstoff-Druck von 50 psi über Platindioxid als Katalysator erschöpfend hydriert. Nach Absaugen des Katalysators wird die verbleibende Lösung unter Vakuum zur Trockne eingeengt. Der Rückstand wird mit Aceton zur Kristallisation gebracht und abgesaugt.
Ausbeute: 62,8 g (95,5 % der Theorie) sehr hygroskopischer Kristalle, die an der Luft zerfließen,
$R_f$-Wert: 0,22 (Kieselgel; Methylenchlorid/Methanol = 9:1)

c) 4-(4-Piperidinyl)-buttersäure-hydrochlorid

Eine Lösung von 62 g (0,201 Mol) 2-[2-(4-Piperidinyl)-ethyl]malonsäure-diethylester-hydrochlorid in 600 ml konzentrierter Salzsäure wird während 24 Stunden auf Rückflußtemperatur erhitzt und anschließend unter Vakuum zur Trockne eingeengt. Der Rückstand wird mit Toluol versetzt und eingeengt. Diese Operation wird noch dreimal wiederholt.
Ausbeute: 44,3 g farblose Kristalle, die noch etwas Toluol enthalten,
$R_f$-Wert: 0,19 (Kieselgel; Methylenchlorid/Methanol = 9:1)

c) 4-(4-Piperidinyl)-buttersäure-methylester-hydrochlorid

In 800 ml Methanol werden unter Rühren bei -10°C langsam 18 ml (0,242 Mol) Thionylchlorid eingetropft. Anschließend tropft man bei gleicher Temperatur eine Lösung von 44,3 g(0,201 Mol) 4-(4-Piperidinyl)-buttersäure-hydrochlorid in 100 ml Methanol zu, rührt über Nacht bei Raumtemperatur weiter und engt dann unter Vakuum zur Trockne ein. Der Rückstand wird zwischen 50%iger Kaliumcarbonat-Lösung und Ether verteilt. Die wäßrige Phase wird noch zweimal mit Ether extrahiert. Die vereinigten Ether-Extrakte werden getrocknet und eingeengt. Der Rückstand wird in Methanol gelöst, mit etherischer Salzsäure bis pH 6 angesäuert und unter Vakuum zur Trockne eingeengt. Der verbleibende Rückstand wird mit Aceton verrieben. Die ausgeschiedenen Kristalle werden abgesaugt.

Auswaage: 35,5 g (88,7 % der Theorie)
Schmelzpunkt: 99-105°C (Zers.)

Beispiel XVII

4-Piperidinyloxyessigsäure-methylester-hydrochlorid

a) N-tert.Butyloxycarbonyl-4-piperidinyloxyessigsäure-methylester

Zu einer Lösung von 10 g (0,05 Mol) N-tert.Butyloxycarbonyl-4-piperidinol in 100 ml trockenen Tetrahydrofuran gibt man unter Rühren 2,3 g (0,05 Mol) Natriumhydrid (50%ig in Öl) und rührt weitere 2 Stunden. Anschließend tropft man unter weiterem Rühren 7,6 g (0,05 Mol) Bromessigsäure-methylester (5 ml) zu und rührt über Nacht weiter. Das nicht umgesetzte Natriumhydrid wird durch Zugabe von Wasser zerstört. Man extrahiert mit Essigester, trocknet die vereinigten Essigester-Extrakte und engt diese unter Vakuum zur Trockne ein. Der Rückstand wird über eine Kieselgel-Säule gereinigt (Elutionsmittel: Methylenchlorid, das 1 % Methanol enthält).
Ausbeute: 4,9 g (36,1 % der Theorie),
Massenspektrum: $M^+$ = 273
$R_f$-Wert: 0,50 (Kieselgel; Methylenchlorid/Methanol = 9,5:0,5)

b) 4-Piperidinyloxyessigsäure-methylester-hydrochlorid

Eine Lösung von 4,9 g (0,018 Mol) N-tert.Butyloxycarbonyl-4-piperidinyloxyessigsäure-methylester in 10 ml Methanol wird mit 30 ml etherische Salzsäure versetzt und während 4 Stunden bei Raumtemperatur stehen gelassen. Anschließend engt man unter Vakuum zur Trockne ein, versetzt den Rückstand mit Ether und saugt den Festkörper ab.
Ausbeute: 3,1 g farbloser Festkörper (82,5 % der Theorie),
Massenspektrum: $M^+$ = 173
$R_f$-Wert: 0,10 (Kieselgel; Methylenchlorid/Mehtanol = 9:1)

Beispiel XVIII

α-Brom-4-methoxycarbonylmethyloxy-acetophenon

a) 4-Methoxycarbonylmethyloxy-acetophenon

Zu einer Lösung von 8 g (0,06 Mol) 4-Hydroxy-acetophenon in 100 ml trockenem Dimethylformamid gibt man 9 g (0,06 Mol) Bromessigsäure-methylester (5,6 ml) und 8 g (0,06 Mol) Kaliumcarbonat. Man erhitzt 5 Stunden lang auf Rückfluß-Temperatur und rührt anschließend über Nacht bei Raumtemperatur. Die Lösung wird unter Vakuum zur Trockne eingeengt und der Rückstand zwischen Essigester und Wasser verteilt. Die vereinigten organischen Extrakte werden getrocknet und unter Vakuum zur Trockne eingeengt. Der Rückstand wird mit Ether verrieben, abgesaugt und getrocknet.
Ausbeute: 8,6 g amorpher Festkörper (70,3 % der Theorie),
Massenspektrum: $M^+$ = 208
$R_f$-Wert: 0,45 (Kieselgel; Essigester/Cyclohexan = 1:1)

b) α-Brom-4-methoxycarbonylmethyloxy-acetophenon

Zu einer Lösung von 2 g (0,0096 Mol) 4-Methoxycarbonylmethyloxy-acetophenon in 40 ml Ether und 10 ml Dioxan tropft man unter Rühren und bei Raumtemperatur ein Suspension von 0,0106 Mol Bromdioxan (hergestellt aus 1,7 g Brom und 8 ml Dioxan) in Dioxan. Nach beendeter Zugabe rührt man weitere 2 Stunden bei Raumtemperatur und engt anschließend unter Vakuum zur Trockne ein.
Ausbeute: 1,3 g Rohprodukt,
$R_f$-Wert: 0,60 Doppelfleck (Kieselgel; Essigester/Cyclohexan = 1:1)
Analog Beispiel XVIII kann folgende Verbindung hergestellt werden:

(1) 4-[α-Brom-acetyl)-phenylessigsäuremethylester Hergestellt aus 4-Acetyl-phenylessigsäuremethylester und Bromdioxan.

Beispiel XIX

3-Methoxycarbonylmethyloxy-anilin

a) 3-Methoxycarbonylmethyloxy-nitrobenzol

Zu einer Lösung von 9 g (0,065 Mol) m-Nitrophenol in 100 ml trockenem Dimethylformamid gibt man 8,8 g (0,065 Mol) Kaliumcarbonat und rührt 1/2 Stunde bei Raumtemperatur. Anschließend gibt man 10,9 g (0,07 Mol) Bromessigsäure-methylester (6,7 ml) zu und erhitzt 5 Stunden lang auf 80°C. Anschließend engt man die Lösung unter Vakuum zur Trockne ein und verteilt den Rückstand zwischen Essigester und Wasser. Die vereinigten organischen Phasen werden getrocknet und unter Vakuum zur Trockne eingeengt. Der Rückstand wird mit Ether verrieben, abgesaugt und getrocknet.
Ausbeute: 9,2 g (67,3 % der Theorie),
$R_f$-Wert: 0,55 (Kieselgel; Methylenchlorid)

b) 3-Methoxycarbonylmethyloxy-anilin

9,2 g (0,046 Mol) 3-Methoxycarbonylmethoxy-nitrobenzol werden in Methanol über 1,5 g Raney-Nickel bei einem Wasserstoffdruck von 50 psi und bei Raumtemperatur erschöpfend hydriert. Nach Absaugen des Katalysators wird die Lösung eingeengt.
Ausbeute: 7,0 g Öl (88,7 % der Theorie),
$R_f$-Wert: 0,50 (Kieselgel; Essigester/Cyclohexan = 1:1)

Beispiel XX

4-Methyloxycarbonylmethyloxy-anilin

a) 4-Methoxycarbonylmethyloxy-nitrobenzol

Hergestellt aus 4-Nitrophenol, Bromessigsäure-methylester und Cäsiumcarbonat analog Beispiel XIXa.
Ausbeute: 10,4 g (91,2 % der Theorie),
Schmelzpunkt: 86-88°C

b) 4-Methoxycarbonylmethyloxy-anilin

Hergestellt aus 4-Methoxycarbonylmethyloxy-nitrobenzol durch Hydrieren über Raney-Nickel analog Beispiel XIXb.
Ausbeute: 9,5 g Harz (98,4 % der Theorie),
$R_f$-Wert: 0,60 (Kieselgel; Methylenchlorid/Methanol = 9:1)

Beispiel XXI

3-(4-Amino-phenyl)-propionsäure-methylester-hydrochlorid

Eine Lösung von 15 g (0,0991 Mol) 3-(4-Amino-phenyl)-propionsäure in 100 ml Methanol wird unter Rühren und Kühlen mit Methanol/Eis tropfenweise mit 12,96 g (0,11 Mol) Thionylchlorid (7,93 ml) versetzt. Nach beendeter Zugabe rührt man weitere 30 Minuten unter Kühlung und anschließend über Nacht bei Raumtemperatur. Anschließend engt man unter Vakuum zur Trockne ein und kristallisiert den Rückstand aus Methanol/Ether.
Ausbeute: 16,8 g (85,6 % der Theorie),
Schmelzpunkt: 165-167°C

Beispiel XXII

N-[[4-(4-Pyridyl)-piperazin-1-yl]carbonyl]piperidin-4-carbonsäure

a) N-[[4-(4-Pyridyl)-piperazin-1-yl]carbonyl]piperidin-4-carbonsäure-methylester

Herstellung durch Umsetzen von Piperidin-4-carbonsäure-methylester mit Chlorameisensäure-p-nitro-phenylester in Gegenwart von Triethylamin zu N-(4-Nitrophenyloxycarbonyl)-piperidin-4-carbonsäure-methylester und anschließender Umsetzung dieses Zwischenproduktes mit 1-(4-Pyridyl)-piperazin analog Beispiel IX.

b) N-[[4-(4-Pyridyl)-piperazin-1-yl]carbonyl]piperidin-4-car bonsäure

Herstellung durch Umsetzung von N-[[4-[4-Pyridyl)-piperazin-1-yl]carbonyl]piperidin-4-carbonsäure-methylester mit halbkonzentrierter Salzsäure analog Beispiel 4.

Beispiel XXIII

4-(Ethoxycarbonyl-2-ethyloxy)-piperidin-trifluoracetat

a) 4-(Ethoxycarbonyl-2-ethyloxy)-N-tert.butyloxycarbonylpiperidin

Zu einer Lösung von 10 g (0,0497 Mol) N-tert.Butyloxycarbonyl-4-piperidinol in 20 ml Dioxan gibt man 0,3 g (0,0027 Mol) Kalium-tert.butylat und anschließend tropfenweise und unter Rühren 13,5 ml (0,124 Mol) Acrylsäure-ethylester und erhitzt während 7 Stunden auf Rückflußtemperatur. Nach Rühren über Nacht bei Raumtemperatur wird unter Vakuum zur Trockne eingeengt und der Rückstand zwischen Essigester und Wasser verteilt. Die organische Phase wird getrocknet und unter Vakuum zur Trockne eingeengt. Der Rückstand wird über eine Kieselgel-Säule gereinigt (Elutionsmittel: Cyclohexan/Essigester = 10:3).
Ausbeute: 4,5 g Öl (30 % der Theorie),
$R_f$-Wert: 0,80 (Kieselgel; Methylenchlorid/Methanol = 9:1)

b) 4-(Ethoxycarbonyl-2-ethyloxy)-piperidin-trifluoracetat

4,5 g (0,015 Mol) 4-(Ethoxycarbonyl-2-ethyloxy)-N-tert.butyloxycarbonyl-piperidin werden in einer Mischung aus 30 ml Methylenchlorid und 20 ml Trifluoressigsäure 4 Stunden lang bei Raumtemperatur stehen gelassen. Man engt unter Vakuum zur Trockne ein und erhält 4,5 g eines farblosen Öls. $R_f$-Wert: 0,20 (Kieselgel; Methylenchlorid/Methanol = 9:1)

Beispiel XXIV

4-trans-(L-Alanyl)-amino-cyclohexancarbonsäuremethylestertrifluoracetat

a) 4-trans-(N-tert.Butyloxycarbonyl-L-alanyl)-amino-cyclohexancarbonsäuremethylester

Zu einer Lösung von 2,5 g (0,013 Mol) (N-tert.Butyloxycarbonyl-L-alanin und 3,9 ml (0,028 Mol) Triethylamin in 100 ml trockenem Dimethylformamid gibt man bei -50°C unter Rühren 1,8 ml (0,0145 Mol) Chlorameisensäureisobutylester und rührt eine Stunde lang bei Raumtemperatur weiter. Anschließend gibt man 2,6 g (0,013 Mol) 4-Amino-cyclohexancarbonsäuremethylester-hydrochlorid zu und läßt über Nacht stehen. Nach Einengen und Verteilen des Rückstandes zwischen Wasser und Essigester wird die organische Phase getrocknet und erneut zur Trockne eingeengt. Der Rückstand wird aus Ether/Petrolether kristallisiert.
Ausbeute: 3,47 g (80 % der Theorie),
Schmelzpunkt: 136-137°C
$R_f$-Wert: 0,60 (Kieselgel; Methylenchlorid/Methanol 9:1)

b) 4-trans-(L-Alanyl)-amino-cyclohexancarbonsäuremethylestertrifluoracetat

Hergestellt aus 3,4 g (0,01 Mol) 4-trans-(N-tert.Butyloxycarbonyl-L-alanyl)-amino-cyclohexancarbonsäuremethylester und 50%iger Trifluoressigsäure in Methylenchlorid analog Beispiel XXIIIb.
Ausbeute 6 g öliges Rohprodukt,
$R_f$-Wert: 0,28 (Kieselgel; Methylenchlorid/Methanol = 9:1)

Beispiel XXV

N-(O-Methyl-L-tyrosyl)-4-piperidinyloxyessigsäure-methylestertrifluoracetat

a) N-(tert.Butyloxycarbonyl-O-methyl-L-tyrosyl)-4-piperidinyloxy-essigsäure-methylester

Hergestellt aus N-tert.Butyloxycarbonyl-O-methyl-L-tyrosin, 4-Piperidinyloxyessigsäure-methylester-hydrochlorid, Chlorameisensäureisobutylester und Triethylamin analog Beispiel XXIVa.

b) N-(O-Methyl-L-tyrosyl)-4-piperidinyloxy-essigsäure-methylester-trifluoracetat

Hergestellt aus N-(tert.Butyloxycarbonyl)-O-methyl-L-tyrosyl)-4-piperidinyloxyessigsäure-methylester und 50%iger Trifluoressigsäure in Methylenchlorid analog Beispiel XXIIIb.

Beispiel XXVI

N-(L-Alanyl-4-piperidinyloxyessigsäure-methylester-trifluoracetat

a) N-(tert.Butyloxycarbonyl-L-alanyl-4-piperidinyloxyessigsäure-methylester

Hergestellt aus N-tert.Butyloxy-L-alanin, 4-Piperidinyloxyessigsäure-methylester-hydrochlorid, Chlorameisensäureisobutylester und Triethylamin analog Beispiel XXIVa.

b) N-(L-Alanyl)-4-piperidinyloxyessigsäure-methylester-trifluoracetat

Hergestellt aus N-(tert.Butyloxycarbonyl-L-alanyl)-4-piperidinyloxyessigsäure-methylester und 50%iger Trifluoressigsäure in Methylenchlorid analog Beispiel XXIIIb.

Beispiel XXVII

4-trans-(O-Methyl-L-tyrosyl)-amino-cyclohexancarbonsäuremethylester-trifluoracetat

a) 4-trans-(N-tert.Butyloxycarbonyl-O-methyl-L-tyrosyl)-aminocyclohexancarbonsäuremethylester

Hergestellt aus N-tert.Butyloxycarbonyl-O-methyl-L-tyrosin, 4-Amino-cyclohexancarbonsäuremethylester-hydrochlorid, Chlorameisensäureisobutylester und Triethylamin analog Beispiel XXIVa.
Schmelzpunkt: 151-153°C
$R_f$-Wert: 0,7 (Kieselgel; Methylenchlorid/Methanol = 9:1)

b) 4-trans-(O-Methyl-L-tyrosyl)-amino-cyclohexancarbonsäuremethylester-trifluoracetat

Hergestellt aus 4-trans-(N-tert.Butyloxycarbonyl-O-methyl-L-tyrosyl)-amino-cyclohexancarbonsäuremethylester und 50%iger Trifluoressigsäure in Methylenchlorid analog Beispiel XXIIIb.
$R_f$-Wert: 0,4 (Kieselgel; Methylenchlorid/Methanol = 9:1)

Beispiel XXVIII

4-Methoxycarbonylmethyloxy-phenylessigsäure

a) 4-Methoxycarbonylmethyloxy-phenylessigsäurebenzylester

Zu einer Suspension von 8,4 g (0,035 Mol) 4-Hydroxy-phenylessigsäurebenzylester und 4,8 g (0,035 Mol) getrocknetem Kaliumcarbonat in 100 ml Dimethylformamid gibt man nach 45 Minuten Rühren bei Raumtemperatur langsam 5,3 g (0,038 Mol) Bromessigsäuremethylester und erhitzt ausschließend unter weiterem Rühren während 5 Minuten auf 80°C. Hiernach wird über Nacht bei Raumtemperatur weiter gerührt. Man filtriert vom Festkörper ab und engt die Mutterlauge unter vermindertem Druck zur Trockne ein. Der Rückstand wird über eine Kieselgel-Säule gereinigt, wobei Methylenchlorid als Elutionsmittel dient.
Ausbeute: 7,9 g amorpher Festkörper (72,9 % der Theorie).

b) 4-Methoxycarbonylmethyloxy-phenylessigsäure

7,8 g (0,025 Mol) 4-Methoxycarbonylmethyloxy-phenylessigsäurebenzylester werden in 150 ml Methanol in Gegenwart von 8 g Palladiumhydroxid auf Kohle bei Raumtemperatur und unter einem Wasserstoffdruck von 50 psi erschöpfend hydriert. Nach Entfernen des Katalysators wird die Mutterlauge unter reduziertem Druck zur Trockne eingeengt.
Ausbeute: 4,7 g harziges Rohprodukt(84,5 % der Theorie).

Beispiel XXIX

3-[[4-Methoxycarbonylmethyl)-piperidinyl]propionsäure-hydrochlorid

a) 3-[[4-Methoxycarbonylmethyl)-piperidinyl]propionsäuretert.butylester

Hergestellt aus 4-Piperidinyl-essigsäuremethylester-hydrochloid, Acrylsäure-tert.butylester und Triton B analog Beispiel 7.

b) 3-[[4-Methoxycarbonylmethyl)-piperidinyl]propionsäurehydrochlorid

Hergestellt aus 3-[(4-Methoxycarbonylmethyl)-piperidinyl]propionsäure-tert.butylester und 50%iger Trifluoressigsäure in Methylenchlorid analog Beispiel XXIIIb.

Beispiel XXX

3-[4-(4-Pyridyl)-piperazin-1-yl]propionsäure

a) 3-[4-[4-(4-Pyridyl)-piperazin-1-yl]propionsäureethylester

Hergestellt aus (4-Pyridyl)-piperazin und Acrylsäureethylester analog Beispiel 7.
$R_f$-Wert: 0,40 (Kieselgel; Methylenchlorid/Methanol = 9:1)

b) 3-[4-(4-Pyridyl)-piperazin-1-yl]propionsäure

Hergestellt aus Verbindung des Beispiels XXXa durch Hydrolyse in Gegenwart von Chlorwasserstoff/Wasser = 1:1 analog Beispiel 5.
$R_f$-Wert: 0,45 (Kieselgel; Methylenchlorid/Methanol/konz.Ammoniak = 4:1:0,25)

Beispiel XXXI

4-((4-Pyridyl)-piperazin-1-yl)oxalsäure

a) 4-((4-Pyridyl)-piperazin-1-yl)oxalsäuremethylester

Hergestellt aus äquimolaren Mengen 4-(4-Pyridyl)-piperazin, Triethylamin und Oxalsäuremethylesterchlorid in Tetrahydrofuran.

b) 4-((4-Pyridyl)-piperazin-1-yl)oxalsäure

Hergestellt aus 4-((4-Pyridyl)-piperazin-1-yl)oxalsäuremethylester durch Hydrolyse mit einer äquimolaren Menge wässriger 1N Natronlauge in Tetrahydrofuran bei Raumtemperatur.
Herstellung der Endprodukte:

Beispiel 1

[4-trans-[3-[4-(4-Pyridyl)-piperazin-1-yl]propionyl]amino]cyclohexancarbonsäure-methylester

Zu einer Lösung von 1,2 g (0,0051 Mol) 3-[4-(4-Pyridyl)-piperazin-1-yl]propionsäure in 100 ml trockenem Dimethylformamid gibt man unter Rühren und bei Raumtemperatur 1,8 g (0,0056 Mol) 2-(1H-Benzotriazol-1-yl)-1,1,3,3-tetramethyluronium-tetrafluorborat, 0,8 g (0,0056 Mol) 1-Hydroxy-1H-benzotriazol, 1 g (0,0051 Mol) p-trans-Aminocyclohexylcarbonsäure-methylesterhydrochlorid und 1 g (0,01 Mol) N-Methyl-morpholin und rührt während 24 Stunden

bei Raumtemperatur weiter. Anschließend engt man unter Vakuum zur Trockene ein. Der verbleibende Rückstand wird mittels Chromatographie über Kieselgel gereinigt (Elutionsmittel: Methylenchlorid/Methanol/konz. Ammoniak = 20:1:0,25).
Ausbeute: 1,1 g (57,6 % der Theorie),
Schmelzpunkt: 162-164°C
$R_f$-Wert: 0,43 (Kieselgel; Methylenchlorid/Methanol/konz. Ammoniak = 9:1:0,25)
Analog Beispiel 1 können folgende Verbindungen hergestellt werden:

(1) N-[3-[4-(4-Pyridyl)-piperazin-1-yl]propionyl]-3-(4-piperidinyl)-propionsäure-methylester-dihydrochlorid

Hergestellt aus 3-[4-(4-Pyridyl)-piperazin-1-yl]propionsäure und 3-(4-Piperidinyl)-propionsäure-methylester. Amorpher Festkörper.
Massenspektrum: $M^+$ = 388
$R_f$-Wert: 0,70 (Kieselgel; Methylenchlorid/Methanol/konz. Ammoniak = 4:1:0,2)

(2) N-[4-[4-(4-Pyridyl)-piperazin-1-yl]butyryl]-4-piperidinylessigsäure-methylester-dihydrochlorid

Hergestellt aus 4-[4-(4-Pyridyl)-piperazin-1-yl]buttersäuredihydrochlorid und 4-Piperidinylessigsäure-methylester-hydrochlorid.
Amorph.
Massenspektrum: $M^+$ = 388
$R_f$-Wert: 0,70 (Kieselgel; Methylenchlorid/Methanol/konz. Ammoniak = 4:1:0,2)

(3) [4-trans-[4-(4-Pyridyl)-piperazin-1-yl]butyrylamino]cyclohexancarbonsäure-methylester-dihydrochlorid

Hergestellt aus 4-[4-(4-Pyridyl)-piperazin-1-yl]buttersäuredihydrochlorid und p-trans-Amino-cyclohexancarbonsäure-methylester-hydrochlorid.
Massenspektrum: $M^+$ = 388
$R_f$-Wert: 0,70 (Kieselgel; Methylenchlorid/Methanol/konz. Ammoniak = 4:1:0,2)

(4) N-[5-[4-(4-Pyridyl)-piperazin-1-yl]valeryl]-4-piperidinylessigsäure-methylester-dihydrochlorid

Hergestellt aus 5-[4-(4-Pyridyl)-piperazin-1-yl]valeriansäuredihydrochlorid und 4-Piperidinylessigsäure-methylester-hydrochlorid.
Amorpher Festkörper.
Massenspektrum: $M^+$ = 402
$R_f$-Wert: 0,75 (Kieselgel; Methylenchlorid/Methanol/konz. Ammoniak = 4:1:0,2)

(5) [4-trans-[5-(4-Pyridyl)-piperazin-1-yl]valerylamino]cyclohexancarbonsäure-methylester-dihydrochlorid

Hergestellt aus 5-[4-(4-Pyridyl)-piperazin-1-yl]valeriansäuredihydrochlorid und p-trans-Amino-cyclohexancarbonsäure-methylester-hydrochlorid.
Amorpher Festkörper.
Massenspektrum: $M^+$ = 402
$R_f$-Wert: 0,75 (Kieselgel; Methylenchlorid/Methanol/konz. Ammoniak = 4:1:0,2)

(6) [4-trans-[4-(4-Pyridyl)-piperazin-1-yl]malonylamino]cyclohexylcarbonsäure-methylester-hydrochlorid

Hergestellt aus 4-[(4-Pyridyl)-piperazin-1-yl]malonsäure-hydrochlorid und p-trans-Amino-cyclohexancarbonsäure-ethylester. Amorpher Festkörper.
Massenspektrum: $M^+$ = 402
$R_f$-Wert: 0,50 (Kieselgel; Methylenchlorid/Methanol/konz. Ammoniak = 9:1:0,1)

(7) [N-[4-(4-Pyridyl)-piperazin-1-yl]-acetyl]-piperazinoessigsäure-methylester-trihydrochlorid

Hergestellt aus [4-(4-Pyridyl)-piperazin-1-yl]essigsäure und Piperazinoessigsäure-methylester-dihydrochlorid.
Schmelzpunkt: 122-123°C
Massenspektrum: $M^+$ = 361
$R_f$-Wert: 0,42 (Kieselgel; Methylenchlorid/Methanol/konz. Ammoniak = 9:1:0,1)

(8) [4-trans-[[4-(4-Pyridyl)-piperazin-1-yl]acetyl]amino]cyclohexancarbonsäure-methylester-dihydrochlorid

Hergestellt aus [4-(4-Pyridyl)-piperazin-1-yl]essigsäure und 4-trans-Amino-cyclohexancarbonsäure-methylester-hydrochlorid.
Schmelzpunkt: 310-313°C
Massenspektrum: $M^+$ = 360
$R_f$-Wert: 0,45 (Kieselgel; Methylenchlorid/Methanol = 9:1)

(9) 1-[[4-(4-Pyridyl)-piperazin-1-yl]carbonyl]-[(piperidin-4-yl)carbonyl]glycin-methylester-hydrochlorid

Hergestellt aus N-[[4-(4-Pyridyl)-piperazin-1-yl]carbonyl]piperazin-4-carbonsäure und Glycin-methylester-hydrochlorid.

(10) N-[[4-(4-Pyridyl)-piperazin-1-yl]acetyl]-4-(4-piperidinyl)-buttersäure-methylester-dihydrochlorid

Hergestellt aus [4-(4-Pyridyl)-piperazin-1-yl]essigsäure und 4-(4-Piperidinyl)-buttersäure-methylester.
Schaum
Massenspektrum: $M^+$ = 388
$R_f$-Wert: 0,48 (Kieselgel; Methylenchlorid/Methanol = 9:1)

(11) [4-trans-[[4-(4-Pyridyl)-piperazin-1-yl]-acetyl]methylamino]-cyclohexancarbonsäure-methylester-dihydrochlorid

Hergestellt aus [4-(4-Pyridyl)-piperazin-1-yl]-essigsäure und 4-trans-Methylamino-cyclohexancarbonsäure-methylester-hydrochlorid.

(12) N-[[4-(4-Pyridyl)-piperazin-1-yl]-carbonylethyl]-4-(piperidinylessigsäuremethylester)-hydrochlorid

Hergestellt aus 1-(4-Pyridyl)-piperazin und 3-[(4-Methoxycarbonylmethyl)-piperidinyl]propionsäure-hydrochlorid.
$R_f$-Wert: 0,21 (Kieselgel; Methylenchlorid/Methanol/konz.Ammoniak = 9:1:0,1)
Massenspektrum: $M^+$ = 374

(13) N-[[4-(4-Pyridyl)-piperazin-1-yl]-malonyl]-4-(piperidinylessigsäuremethylester)-hydrochlorid

Hergestellt aus 4-[(4-Pyridyl)-piperazin-1-yl]-malonsäure und 4-Piperidinylessigsäuremethylester-hydrochlorid.
$R_f$-Wert: 0,48 (Kieselgel; Methylenchlorid/Methanol/konz.Ammoniak = 9:1:0,1)
Massenspektrum: $M^+$ = 394

(14) [4-trans-[(4-Pyridyl)-piperazin-1-yl]-oxalylamino]-cyclohexancarbonsäuremethylester

Hergestellt aus 4-[(4-Pyridyl)-piperazin-1-yl]oxalsäure und trans-4-Aminocyclohexancarbonsäuremethylester-hydrochlorid.

(15) N-[[4-(4-Pyridyl)-piperazin-1-yl]-oxalyl]-4-[piperidinylessigsäuremethylester

Hergestellt aus 4-[(4-Pyridyl)-piperazin-1-yl]oxalsäure und 4-Piperidinylessigsäuremethylester-hydrochlorid.

(16) N-[[4-(4-Pyridyl)-piperazin-1-yl]-ethylcarbonyl]-4-[piperidinylessigsäuremethylester-hydrochlorid

Hergestellt aus 3-[4-(4-Pyridyl)-piperazin-1-yl]propionsäure und 4-Piperidinylessigsäuremethylester-hydrochlorid.
$R_f$-Wert: 0,38 (Kieselgel; Methylenchlorid/Methanol/konz.Ammoniak = 9:1:0,1)
Massenspektrum: $M^+$ = 374

Beispiel 2

N-[3-[4-(4-Pyridyl)-piperazin-1-yl]propionyl]-4-piperidinylessigsäure-methylester-dihydrochlorid

Zu einer Lösung von 0,3 g (0,0013 Mol) 3-[4-(4-Pyridyl)-piperazin-1-yl]propionsäure in 30 ml trocknem Dimethylformamid gibt man unter Rühren und bei Raumtemperatur 0,26 g (0,0013 Mol) N,N'-Dicyclohexyl-carbodiimid, 0,2 g (0,0013 Mol) 1-Hydroxy-1H-benzotriazol und 0,25 g (0,0013 Mol) 4-Piperidino-essigsäuremethylester und rührt während

24 Stunden bei Raumtemperatur weiter. Anschließend engt man unter Vakuum zur Trockne ein. Der verbleibende Rückstand wird mittels Chromatographie über Kieselgel gereinigt (Elutionsmittel: Methylenchlorid, das 6 %, 8 % und 10 % Methanol enthält). Der nach dem Eindampfen verbleibende Rückstand wird in Methanol gelöst. Diese Lösung wird mit ätherischer Salzsäure bis pH 3 angesäuert und unter Vakuum zur Trockne eingeengt.
Ausbeute: 0,6 g (37,7 % der Theorie), amorpher Festkörper $R_f$-Wert: 0,48 (Kieselgel; Methylenchlorid/Methanol = 4:1)

Beispiel 3

[4-[3-[4-(4-Pyridyl)-piperazin-1-yl]propionyl]amino]phenylessigsäuremethylester-dihydrochlorid

Man läßt eine Lösung von 3,1 g (0,01 Mol) 3-[4-(4-Pyridyl)-piperazin-1-yl]propionsäure, 1,8 g (0,011 Mol) N,N'-Carbonyldiimidazol, 3 g (0,03 Mol) N-Methyl-morpholin und 2 g (0,01 Mol) 4-Aminophenylessigsäure-methylester in 200 ml trockenem Dimethylformamid über Nacht bei Raumtemperatur stehen und engt anschließend unter Vakuum zur Trockne ein. Der verbleibende Rückstand wird mittels Chromatographie über Kieselgel gereinigt (Elutionsmittel: Methylenchlorid/Methanol/konz. Ammoniak = 9:1:0,1). Nach Eindampfen der Eluate wird der verbleibende Rückstand in Methanol gelöst und mit etherischer Salzsäure bis pH 3 angesäuert. Man engt erneut unter Vakuum zur Trockne ein und erhält einen amorphen farblosen Festkörper.
Ausbeute: 1,0 g (22 % der Theorie),
Massenspektrum: $M^+$ = 382
$R_f$-Wert: 0,75 (Kieselgel; Methylenchlorid/Methanol/konz. Ammoniak = 4:1:0,2)

Beispiel 4

3-[4-trans-[4-(4-Pyridyl)-piperazin-1-yl]carbonylamino]cyclohexylpropionsäure-methylester

Zu einer Lösung von 1,2 g (0,007 Mol) N,N'-Carbonyldiimidazol und 0,6 g (0,0092 Mol) Imidazol in 50 ml trockenem Tetrahydrofuran gibt man bei 0°C unter Rühren eine Suspension von 1,4 g (0,0061 Mol) p-trans-Amino-cyclohexylpropionsäure-methylester und anschließend 0,8 g (0,0061 Mol) N-Ethyl-diisopropylamin. Nach beendeter Zugabe wird während einer halben Stunde bei +5°C gerührt und dann eine Lösung von 1 g (0,0061 Mol) 4-Pyridylpiperazin in 30 ml trockenem Tetrahydrofuran zugetropft. Nach beendeter Zugabe rührt man über Nacht bei Raumtemperatur. Die Lösung wird unter Vakuum zur Trockne eingeengt und der verbleibende Rückstand zwischen Essigester und Wasser verteilt. Die vereinigten Essigester-Phasen werden getrocknet und eingedampft. Der Rückstand wird durch Chromatographie über eine Kieselgel-Säule gereinigt (Elutionsmittel: Methylenchlorid/Methanol/konz. Ammoniak = 9:1:0,1).
Ausbeute: 0,5 g (21,8 % der Theorie),
Schmelzpunkt: 64-66°C
Massenspektrum: $M^+$ = 374
$R_f$-Wert: 0,7 (Kieselgel; Methylenchlorid/Methanol/konz. Ammoniak = 4:1:0,2)
Analog Beispiel 4 können folgende Verbindungen hergestellt werden:

(1) 4-[[4-(4-Pyridyl)-piperazin-1-yl]carbonylamino]piperidinoessigsäure-methylester

Hergestellt aus 4-Pyridyl-piperazin, 4-Amino-piperidinoessigsäure-methylester, Imidazol und N,N'-Carbonyldiimidazol.
Schmelzpunkt: 143-144°C
Massenspektrum: $M^+$ = 361
$R_f$-Wert: 0,45 (Kieselgel; Methylenchlorid/Methanol/konz. Ammoniak = 9:1:0,1)

(2) [3-[[4-(4-Pyridyl)-piperazin-1-yl]carbonylamino]phenoxy]essigsäure-methylester

Hergestellt aus 1-(4-Pyridyl)-piperazin und 3-Methoxycarbonylmethyloxy-anilin mit 1,1'-Carbonyldi-(1,2,4-triazol).
Schmelzpunkt: 180-182°C
Massenspektrum: $M^+$ = 370
$R_f$-Wert: 0,37 (Kieselgel; Methylenchlorid/Methanol/konz. Ammoniak = 9:1:0,1)

Beispiel 5

[4-trans-[3-[4-(4-Pyridyl)-piperazin-1-yl]propionyl]amino]-cyclohexancarbonsäure-dihydrochlorid

Eine Lösung von 0,4 g (0,0011 Mol) [4-trans-[3-[4-(4-Pyridyl)piperazin-1-yl]propionyl]amino]cyclohexancarbonsäure-methylester in 40 ml halbkonzentrierter Salzsäure wird über Nacht bei Raumtemperatur gerührt und anschließend unter Vakuum zur Trockne eingeengt. Der verbleibende Rückstand wird mit Aceton verrieben, der Festkörper abfiltriert, mit Aceton gewaschen und getrocknet.
Ausbeute: 0,35 g (90,9 % der Theorie),
Schmelzpunkt: 252-254°C
$R_f$-Wert: 0,24 (Kieselgel; Methylenchlorid/Methanol/konz. Ammoniak = 4:1:0,25)
Analog Beispiel 5 können folgende Verbindungen hergestellt werden:

(1) N-[3-[4-(4-Pyridyl)-piperazin-1-yl]propionyl]-4-piperidinyl-essigsäure-dihydrochlorid

Hergestellt aus N-[3-[4-(4-Pyridyl)-piperazino]propionyl]-4-piperidinylessigsäure-methylester-dihydrochlorid
Schmelzpunkt: 218-220°C
$R_f$-Wert: 0,27 (Kieselgel; Methylenchlorid/Methanol = 4:1)

(2) N-[3-[4-(4-Pyridyl)-piperazin-1-yl]propionyl]-3-(4-piperidinyl)-propionsäure

Hergestellt aus N-[3-[4-(4-Pyridyl)-piperazin-1-yl]propionyl]-3-(4-piperidinyl)-propionsäure-methylester-dihydrochlorid Amorpher Festkörper.
Massenspektrum: $M^+ = 374$
$R_f$-Wert: 0,22 (Kieselgel; Methylenchlorid/Methanol/konz. Ammoniak = 4:1:0,2)

(3) N-[4-[4-(4-Pyridyl)-piperazin-1-yl]butyryl]-4-piperidinylessigsäure-dihydrochlorid

Hergestellt aus N-[4-[4-(4-Pyridyl)-piperazin-1-yl]-butyryl]-4-piperidinylessigsäure-methylester-dihydrochlorid
Amorpher Festkörper.
Massenspektrum: $(M+H)^+ = 375$
$R_f$-Wert: 0,13 (Kieselgel; Methylenchlorid/Methanol/konz. Ammoniak = 4:1:0,2)

(4) [4-trans-[4-[4-(4-Pyridyl)-piperazin-1-yl]butyryl]amino]cyclohexancarbonsäure-dihydrochlorid

Hergestellt aus [4-trans-[4-[4-(4-Pyridyl)-piperazin-1-yl]butyryl]amino]cyclohexancarbonsäure-methylester-dihydrochlorid. Amorpher Festkörper.
Massenspektrum: $(M+H)^+ = 375$
$R_f$-Wert: 0,19 (Kieselgel; Methylenchlorid/Methanol/konz. Ammoniak = 4:1:0,2)

(5) [4-trans-[5-[4-(4-Pyridyl)-piperazin-1-yl]valeryl]amino]cyclohexancarbonsäure-dihydrochlorid

Hergestellt aus [4-trans-[5-[4-(4-Pyridyl)-piperazin-1-yl]-valeryl]amino]cyclohexancarbonsäure-methylester-dihydrochlorid.
Massenspektrum: $M^+ = 388$
$R_f$-Wert: 0,18 (Kieselgel; Methylenchlorid/Methanol/konz. Ammoniak = 4:1:0,2)

(6) 3-[4-trans-[4-(4-Pyridyl)-piperazin-1-yl]carbonylamino]cyclohexylpropionsäure-hydrochlorid

Hergestellt aus 3-[4-trans-[4-(4-Pyridyl)-piperazin-1-yl]-carbonylamino]cyclohexylpropionsäure-methylester.
Schmelzpunkt: 140-142°C
Massenspektrum: $M^+ = 360$
$R_f$-Wert: 0,10 (Kieselgel; Methylenchlorid/Methanol/konz. Ammoniak = 4:1:0,2)

(7) [4-trans-[4-(4-Pyridyl)-piperazin-1-yl]malonylamino]cyclohexylcarbonsäure-hydrochlorid

Hergestellt aus [4-trans-[4-(4-Pyridyl)-piperazin-1-yl]malonylamino]cyclohexylcarbonsäure-ethylester-hydrochlorid.
Schmelzpunkt: 203-205°C

Massenspektrum: $M^+$ = 360
$R_f$-Wert: 0,10 (Kieselgel; Methylenchlorid/Methanol/konz. Ammoniak = 9:1:0,1)

(8) <u>3-[4-[4-(4-Pyridyl)-piperazin-1-yl]carbonylamino]piperidinopropionsäure-dihydrochlorid</u>

Hergestellt aus 3-[4-[4-(4-Pyridyl)-piperazin-1-yl]-carbonylamino]piperidinopropionsäure-methylester.
Schmelzpunkt: 235-236°C
Massenspektrum: $M^+$ = 361
$R_f$-Wert: 0,11 (Kieselgel; Methylenchlorid/Methanol/konz. Ammoniak = 4:1:0,2)

(9) <u>[4-[4-(4-Pyridyl)-piperazin-1-yl]carbonylamino]piperidinoessigsäure</u>

Hergestellt aus [4-[4-(4-Pyridyl)-piperazin-1-yl]carbonylamino]piperidinoessigsäure-methylester.
Schmelzpunkt: 210-212°C
Massenspektrum: $M^+$ = 347
$R_f$-Wert: 0,10 (Kieselgel; Methylenchlorid/Methanol/konz. Ammoniak = 4:1:0,2)

(10) <u>N-[[4-(4-Pyridyl)-piperazin-1-yl]carbonyl]-3-(4-piperidinyl)-propionsäure-hydrochlorid</u>

Hergestellt aus N-[[4-(Pyridyl)-piperazin-1-yl]carbonyl]-3-(4-piperidinyl)-propionsäure-methylester-hydrochlorid.
Schmelzpunkt: 171-178°C
$R_f$-Wert: 0,47 (Reversed Phase Platte RP18; Methanol/Natriumchlorid-Lösung (5%ig) = 6:4)

(11) <u>N-[4-[[4-(4-Pyridyl)-piperazin-1-yl]carbonyl]piperidinyl]β-alanin-dihydrochlorid</u>

Hergestellt aus N-[4-[[4-(Pyridyl)-piperazin-1-yl]carbonyl]-piperidinyl]-β-alanin-methylester-dihydrochlorid.
Schmelzpunkt: 234-237°C
$R_f$-Wert: 0,75 (Reversed Phase Platte RP18; Methanol/5%ige Natriumchlorid-Lösung = 6:4)

(12) <u>N-Methyl-N-[4-[[4-(4-pyridyl)-piperazin-1-yl]carbonyl]piperidinyl[-β-alanin-dihydrochlorid</u>

Hergestellt aus N-Methyl-N-[4-[[4-(4-pyridyl)-piperazin-1-yl]carbonyl]piperidinyl]-β-alanin-methylester.

(13) <u>N-Acetyl-N-[4-[[4-(4-pyridyl)-piperazin-1-yl]carbonyl]piperidinyl]-β-alanin-hydrochlorid</u>

Hergestellt aus N-Acetyl-N-[4-[[4-(4-pyridyl)-piperazin-1-yl]carbonyl]piperidinyl]-β-alanin-methylester-hydrochlorid.

(14) <u>N-[4-[[4-(4-Pyridyl)-piperazin-1-yl]carbonyl]piperidinyl]glycin-dihydrochlorid</u>

Hergestellt aus N-[4-[[4-(4-Pyridyl)-piperazin-1-yl]carbonyl]piperidinyl]glycin-methylester-dihydrochlorid.

(15) <u>N-Methyl-N-[4-[[4-(4-pyridyl)-piperazin-1-yl]carbonyl]piperidinyl]glycin-dihydrochlorid</u>

Hergestellt aus N-Methyl-N-[4-[[4-(4-pyridyl)-piperazin-1-yl]carbonyl]piperidinyl]glycin-methylester-dihydrochlorid.

(16) <u>N-Acetyl-N-[4-[[4-(4-pyridyl)-piperazin-1-yl]carbonyl]piperidinyl]glycin-hydrochlorid</u>

Hergestellt aus N-Acetyl-N-[4-[[4-(4-pyridyl)-piperazin-1-yl]carbonyl]piperidinyl]glycin-methylester-hydrochlorid.

(17) <u>N-(2-Phenylethyl)-N-[4-[[4-(4-pyridyl)-piperazin-1-yl]carbonyl]piperidinyl]-β-alanin-dihydrochlorid</u>

Hergestellt aus N-(2-Phenylethyl)-N-[4-[[4-(4-pyridyl)-piperazin-1-yl]carbonyl]piperidinyl]-β-alanin-methylester.

(18) <u>N-[[4-(4-Pyridyl)-piperazin-1-yl]carbonyl]-4-(4-piperidinyl)-buttersäure-hydrochlorid</u>

Hergestellt aus N-[[4-(4-Pyridyl)-piperazin-1-yl]-carbonyl]-4-(4-piperidinyl)-buttersäure-methylester-hydrochlorid.
Schmelzpunkt: 147-149°C (Zers.)
$R_f$-Wert: 0,29 (Reversed Phase Platte RP18; Methanol/5%ige Natriumchlorid-Lösung = 6:4)

(19) N-[[4-(4-Pyridyl)-piperazin-1-yl]acetyl]piperazinoessigsäure-trihydrochlorid

Hergestellt aus N-[[4-(4-Pyridyl)-piperazin-1-yl]acetyl]piperazinoessigsäure-methylester-trihydrochlorid.

(20) [4-trans-[[4-(4-Pyridyl)-piperazin-1-yl]acetyl]amino]cyclohexancarbonsäure-dihydrochlorid

Hergestellt aus [4-trans-[[4-(4-Pyridyl)-piperazin-1-yl]acetyl]amino]cyclohexancarbonsäure-methylester-dihydrochlorid.
Schmelzpunkt: 298-300°C (Zers.)
Massenspektrum: $M^+ = 346$
$R_f$-Wert: 0,70 (Reversed Phase Platte RP18; Methanol/5%ige Natriumchlorid-Lösung = 6:4)

(21) [3-[[4-(4-Pyridyl)-piperazin-1-yl]carbonylamino]phenoxy]essigsäure-hydrochlorid

Hergestellt aus [3-[[4-(4-Pyridyl)-piperazin-1-yl]carbonylamino]phenoxy]essigsäure-methylester.
Schmelzpunkt: 225-228°C
Massenspektrum: $M^+ = 356$
$R_f$-Wert: 0,60 (Kieselgel; Methylenchlorid/Methanol/konz. Ammoniak = 2:1:0,25)

(22) 4-[[4-(4-Pyridyl)-piperazin-1-yl]acetyl]phenylessigsäuredihydrochlorid

Hergestellt aus 4-[[4-(4-Pyridyl)-piperazin-1-yl]acetyl]phenylessigsäure-methylester-dihydrochlorid.

(23) 3-[4-[4-(4-Pyridyl)-piperazin-1-yl]carbonylamino]phenyl]propionsäure-hydrochlorid

Hergestellt aus 3-[4-[4-(4-Pyridyl)-piperazin-1-yl)carbonylamino]phenyl]propionsäure-methylester-hydrochlorid.

(24) N-[4-trans-[4-(4-Pyridyl)-piperazin-1-yl]carbonylamino]cyclohexyl]glycin-dihydrochlorid

Hergestellt aus N-[4-trans-[4-(4-Pyridyl)-piperazin-1-yl]carbonylamino]cyclohexyl]glycin-methylester-dihydrochlorid.

(25) N-[4-trans-[4-(4-Pyridyl)-piperazin-1-yl]carbonylaminocyclohexyl]sarkosin-dihydrochlorid

Hergestellt aus N-[4-trans-[4-(4-Pyridyl)-piperazin-1-yl]-carbonylamino]cyclohexyl]sarkosin-methylester-dihydrochlorid.

(26) N-Acetyl-N-[4-trans-[4-(4-pyridyl)-piperazin-1-yl]carbonylaminocyclohexyl]glycin-hydrochlorid

Hergestellt aus N-Acetyl-N-[4-trans-[4-(4-pyridyl)-piperazin-1-yl]carbonylaminocyclohexyl]glycin-methylester-hydrochlorid.

(27) N-[4-[4-(4-Pyridyl)-piperazin-1-yl]carbonylaminophenyl]glycin-hydrochlorid

Hergestellt aus N-[4-[4-(4-Pyridyl)-piperazin-1-yl]carbonylaminophenyl]glycin-methylester-hydrochlorid.

(28) N-[4-[4-(4-Pyridyl)-piperazin-1-yl]carbonylaminophenyl]sarkosin-hydrochlorid

Hergestellt aus N-[4-[4-(4-Pyridyl)-piperazin-1-yl]carbonylaminophenyl]sarkosin-methylester-hydrochlorid.

(29) N-Acetyl-N-[4-[4-(4-pyridyl)-piperazin-1-yl]carbonylaminophenyl]glycin-hydrochlorid

Hergestellt aus N-Acetyl-N-[4-[4-(4-pyridyl)-piperazin-1-yl]carbonylaminophenyl]glycin-methylester-hydrochlorid.

(30) N-[[4-(4-Pyridyl)-piperazin-1-yl]carbonyl]-3-(piperidin-4-yloxy)-propionsäure-hydrochlorid

Hergestellt aus N-[[4-(4-Pyridyl)-piperazin-1-yl]-carbonyl]-3-(piperidin-4-yloxy)-propionsäure-ethylester-hydrochlorid. Schmelzpunkt: 118-122°C (Zers.)

(31) [4-trans-[[4-(4-Pyridyl)-piperazin-1-yl]carbonylamino]cyclohexyloxy]essigsäure-hydrochlorid

Hergestellt aus [4-trans-[[4-(4-Pyridyl)-piperazin-1-yl]carbonylamino]cyclohexyloxy]essigsäure-methylester-hydrochlorid.

(32) N-[[4-(4-Pyridyl)-piperazin-1-yl]-carbonyl]-(piperidin-4-yloxy)-essigsäure-hydrochlorid

Hergestellt aus N-[[4-(4-Pyridyl)-piperazin-1-yl]carbonyl]-(piperidin-4-yloxy)-essigsäure-methylester-hydrochlorid.

(33) N-[[4-(4-Pyridyl)-piperazin-1-yl]-carbonyl](piperidin-4-yl)-carbonyl]glycin-hydrochlorid

Hergestellt aus N-[[4-(4-Pyridyl)-piperazin-1-yl]carbonyl]-(piperidin-4-yl)-carbonyl]glycin-methylester-hydrochlorid.

(34) N-[[4-(4-Pyridyl)-piperazin-1-yl]acetyl]-4-(4-piperidinyl)-buttersäure-dihydrochlorid

Hergestellt aus N-[[4-(4-Pyridyl)-piperazin-1-yl]acetyl]-4-(4-piperidinyl)-buttersäure-methylester-dihydrochlorid.
Schmelzpunkt: 109-112°C
Massenspektrum: $M^+$ = 374
$R_f$-Wert: 0,60 (Reversed Phase Platte RP18; Methanol/5ige Natriumchlorid-Lösung = 6:4)

(35) [4-trans-[[4-(4-Pyridyl)-piperazin-1-yl]acetyl]methylamino]-cyclohexancarbonsäure-dihydrochlorid

Hergestellt aus [4-trans-[[4-(4-Pyridyl)-piperazin-1-yl]acetyl]methylamino]cyclohexancarbonsäuremethylester-dihydrochlorid.

(36) [4-trans-[2S-[4-(4-Pyridyl)-piperazin-1-yl]-propionyl]-amino]cyclohexancarbonsäure-dihydrochlorid

Hergestellt aus [4-trans-[2S-[4-(4-Pyridyl)-piperazin-1-yl]propionyl]amino]cyclohexancarbonsäuremethylester-dihydrochlorid.

(37) [4-trans-[2S-[4-(4-Pyridyl)-piperazin-1-yl]-(3-(4-methoxyphenyl)-propionyl)]amino]cyclohexancarbonsäure-dihydrochlorid

Hergestellt aus [4-trans-[2S-[4-(4-Pyridyl)-piperazin-1-yl](3-(4-methoxyphenyl)-propionyl)]amino]cyclohexancarbonsäuremethylester-dihydrochlorid.

(38) N-[2S-[4-(4-Pyridyl)-piperazin-1-yl]-propionyl]piperidin-4-yloxy)-essigsäure-dihydrochlorid

Hergestellt aus N-[2S-[4-(4-Pyridyl)-piperazin-1-yl]propionyl]-(piperidin-4-yloxy)-essigsäuremethylester-dihydrochlorid.

(39) N-[2S-[4-(4-Pyridyl)-piperazin-1-yl]-(3-(4-methoxyphenyl)-propionyl)]piperidin-4-yloxy)-essigsäure-dihydrochlorid

Hergestellt aus N-[2S-[4-(4-Pyridyl)-piperazin-1-yl]-(3-(4-methoxyphenyl)-propionyl)]-(piperidin-4-yloxy)-essigsaure-methylester-dihydrochlorid.

(40) N-[[4-(4-Pyridyl)-piperazin-1-yl]-carbonylethyl]-4-(piperidinylessigsäure)-hydrochlorid

Hergestellt aus N-[[4-(4-Pyridyl)-piperazin-1-yl]carbonylethyl]-4-(piperidinylessigsäuremethylester)-hydrochlorid.
$R_f$-Wert: 0,095 (Kieselgel; Methylenchlorid/Methanol/konz. Ammoniak = 2:1:0,25)
Massenspektrum: $(M+H)^+$ = 361

(41) N-[[4-(4-Pyridyl)-piperazin-1-yl]ethylcarbonyl]-4-(piperidinylessigsäure)-hydrochlorid

Hergestellt aus N-[[4-(4-Pyridyl)-piperazin-1-yl]ethylcarbonyl]-4-(piperidinylessigsäuremethylester)-hydrochlorid.
$R_f$-Wert: 0,5 (Reversed Phase Platte RP18; Methanol/5%ige = NaCl-Lösung = 3:2)
Massenspektrum: $M^+$ = 360

(42) N-[[4-(4-Pyridyl)-piperazin-1-yl]malonyl]-4-(piperidinylessigsäure)-hydrochlorid

Hergestellt aus N-[[4-(4-Pyridyl)-piperazin-1-yl]malonyl]-4-(piperidinylessigsäuremethylester)-hydrochlorid.
Massenspektrum: $M^+ = 374$

(43) [4-trans-[(4-Pyridyl)-piperazin-1-yl]-oxalylamino]cyclohexancarbonsäure-hydrochlorid

Hergestellt aus [4-trans-[(4-Pyridyl)-piperazin-1-yl]oxalylamino]cyclohexancarbonsäuremethylester und äquimolaren Mengen Natriumhydroxid.

(44) N-[[4-(4-Pyridyl)-piperazin-1-yl]-oxalyl]-4-(piperidinylessigsäure)

Hergestellt aus N-[[4-(4-Pyridyl)-piperazin-1-yl]-oxalyl]-4-(piperidinylessigsäuremethylester) und äquimolaren Mengen Natriumhydroxid.

Beispiel 6

4-[3-[4-(4-Pyridyl)-piperazin-1-yl]propionylamino]phenylessigsäure

Man versetzt eine Lösung von 0,6 g (0,0013 Mol) 4-[3-[4-(4-Pyridyl)-piperazin-1-yl]propionylamino]phenylessigsäuremethyl ester-dihydrochlorid in 10 ml Tetrahydrofuran mit 10 ml 1n Natronlauge und rührt über Nacht. Anschließend neutralisiert man mit 2n Salzsäure und engt unter Vakuum zur Trockne ein. Der verbleibende Rückstand wird zweimal mit absolutem Ethanol extrahiert. Die vereinigten Ethanol-Extrakte werden unter Vakuum zur Trockene eingeengt. Den Rückstand extrahiert man mit Methylenchlorid/Methanol = 1:1, engt die vereinigten Extrakte zur Trockne ein und verreibt den Rückstand mit Ether. Der ausgeschiedene amorphe Festkörper wird abgesaugt und getrocknet.
Ausbeute: 0,46 g (94,8 % der Theorie),
Massenspektrum: $(M+H)^+ = 369$
$R_f$-Wert: 0,68 (Kieselgel; Methylenchlorid/Methanol/konz. Ammoniak = 2:1:0,2)
Analog Beispiel 6 wird folgende Verbindung hergestellt:

(1) N-[5-[4-(4-Pyridyl)-piperazin-1-yl]valeryl]-4-piperidinylessigsäure

Hergestellt aus N-[5-[4-(4-Pyridyl)-piperazin-1-yl]-valeryl]-4-piperidinylessigsäure-methylester und 1n Natronlauge.
Amorpher Festkörper.
Massenspektrum: $M^+ = 388$
$R_f$-Wert: 0,28 (Kieselgel; Methylenchlorid/Methanol/konz. Ammoniak = 4:1:0,2)

Beispiel 7

3-[4-[[4-(4-Pyridyl)-piperazin-1-yl]carbonylamino]piperidino]propionsäure-methylester

Zu einer Lösung von 2 g (0,0069 Mol) 4-[[4-(4-Pyridyl)-piperazin-1-yl]carbonylamino]piperidin in 30 ml Methanol gibt man 0,6 g (0,0069 Mol) Acrylsäuremethylester (0,7 ml) und läßt 4 Stunden lang bei Raumtemperatur stehen. Anschließend engt man unter Vakuum zur Trockne ein und reinigt den Rückstand über eine Kieselgelsäule, wobei Methylenchlorid/Methanol/konz. Ammoniak = 9:5:0,05 als Elutionsmittel verwendet wird.
Ausbeute: 1,5 g (77,1 % der Theorie),
Farbloser Festkörper.
Massenspektrum: $M^+ = 375$
$R_f$-Wert: 0,38 (Kieselgel; Methylenchlorid/Methanol/konz. Ammoniak = 9:1:0,1)
Analog Beispiel 7 wird folgende Verbindung hergestellt:

(1) 4-[[4-(4-Pyridyl)-piperazin-1-yl]-ethylcarbonyl]phenoxyessigsäure-methylester-hydrochlorid

Hergestellt aus 1-(4-Pyridyl)-piperazin und 4-Acryloyl-phenoxyessigsäuremethylester.

Beispiel 8

N-[4-(4-Pyridyl)-piperazin-1-yl]carbonyl]-3-(4-piperidinyl)-propionsäuremethylester-hydrochlorid

Ein Gemisch von 9 g (0,0134 Mol) 4-(4-Nitrophenyloxycarbonyl)-3-(4-piperidinyl)-propionsäure-methylester, 2,2 g (0,0134 Mol) 1-(4-Pyridyl)-piperazin und N-Ethyl-diisopropylamin wird 4 Stunden lang auf 140°C erhitzt und nach dem Abkühlen mit Ether verrieben und abdekantiert. Der Festkörper wird zwischen Essigester und Wasser verteilt, die organische Phase mit 1n Natronlauge und anschließend mit Wasser gewaschen, getrocknet und unter Vakuum zur Trockne eingeengt. Der Rückstand wird über eine Kieselgel-Säule gereinigt, wobei Methylenchlorid mit 2,5, 3 und 5 % Methanol als Elutionsmittel dient. Der gelbe Rückstand wird in Ether gelöst. Diese Lösung wird mit etherischer Salzsäure angesäuert. Der ausgeschiedene Festkörper wird abgesaugt.
Ausbeute: 1,5 g (28,2 % der Theorie),
Schmelzpunkt: >320°C
Massenspektrum: $M^+ = 360$
$R_f$-Wert: 0,57 (Kieselgel; Methylenchlorid/Methanol = 9:1)
Analog Beispiel 8 können folgende Verbindungen hergestellt werden:

(1) N-Methyl-N-[4-[[4-(4-pyridyl)-piperazin-1-yl]carbonyl]piperidinyl]-β-alaninmethylester

Hergestellt aus N-Methyl-N-[4-[1-(4-nitrophenyloxycarbonyl)-piperidinyl]-β-alanin-methylester und 1-(4-Pyridyl)-piperazin.

(2) N-Acetyl-N-[4-[[4-(4-pyridyl)-piperazin-1-yl]carbonyl]piperidinyl]-β-alanin-methylester

Hergestellt aus N-Acetyl-N-[4-[1-(4-nitrophenyloxycarbonyl)-piperidinyl]]-β-alanin-methylester und 1-(4-Pyridyl)-piperazin.

(3) N-Methyl-N-[4-[[4-(4-pyridyl)-piperazin-1-yl]carbonyl]piperidinyl]glycin-methylester-dihydrochlorid

Hergestellt aus N-Methyl-N-[4-(1-(4-nitrophenyloxycarbonyl)-piperidinyl]]glycin-methylester und 1-(4-Pyridyl)-piperazin.

(4) N-Acetyl-N-[4-[[4-(4-pyridyl)-piperazin-1-yl]carbonyl]piperidinyl]glycin-methylester

Hergestellt aus N-Acetyl-N-[4-[1-(4-nitrophenyloxycarbonyl)-piperidinyl]]glycin-methylester und 1-(4-Pyridyl)-piperazin.

(5) N-(2-Phenylethyl)-N-[4-[[4-(4-pyridyl)-piperazin-1-yl]carbonyl]piperidinyl]-β-alanin-methylester

Hergestellt aus N-(2-Phenylethyl)-N-[4-[1-(4-nitrophenyloxycarbonyl)-piperidinyl]]-β-alanin-methylester und 1-(4-Pyridyl)-piperazin.

(6) N-[[4-(4-Pyridyl)-piperazin-1-yl]carbonyl]-4-(4-piperidinyl)-buttersäure-methylester-hydrochlorid

Hergestellt aus N-(4-Nitrophenyloxycarbonyl)-4-(4-piperidinyl)-buttersäure-methylester und 1-(4-Pyridyl)-piperazin.
Schmelzpunkt: 112-115°C (Zers.)
$R_f$-Wert: 0,45 (Kieselgel; Methylenchlorid/Methanol = 9:1)

(7) 3-[[4-(4-Pyridyl)-piperazin-1-yl]carbonylamino]phenyl]propionsäure-hydrochlorid

Hergestellt aus 3-[4-[4-Nitrophenyloxycarbonylamino]phenyl]propionsäure-methylester, 1-(4-Pyridyl)-piperazin und Triethylamin.

(8) N-[[4-(4-Pyridyl)-piperazin-1-yl]carbonyl]-3-(piperidin-4-yloxy)-propionsäure-ethylester-hydrochlorid

Hergestellt aus N-(4-Nitro-phenyloxycarbonyl)-(3-piperidin-4-yloxy)-propionsäure-ethylester und 1-(4-Pyridyl)-piperazin. Schmelzpunkt: 105-106°C (Zers.)
Massenspektrum: $M^+ = 390$
$R_f$-Wert: 0,18 (Kieselgel; Methylenchlorid/Methanol = 9:1)

Beispiel 9

N-[4-[[4-(4-Pyridyl)-piperazin-1-yl]carbonyl]piperidinyl]β-alanin-methylester-dihydrochlorid

Zu einer Lösung von 1,6 g (0,0034 Mol) N-tert.Butyloxycarbonyl-N-[4-[[4-(4-pyridyl)-piperazin-1-yl]carbonyl]piperidinyl]-β-alanin-methylester in 20 ml Methylenchlorid gibt man bei Raumtemperatur und unter Rühren 10 ml Trifluoressigsäure und läßt 3 Stunden stehen. Anschließend engt man unter Vakuum ein, versetzt den Rückstand mit Aceton und engt wieder zur Trockne ein. Der Rückstand wird in Methanol gelöst, mit etherischer Salzsäure angesäuert und erneut unter Vakuum zur Trockne eingeengt. Der feste verbleibende Rückstand wird mit Aceton verrieben. Der Festkörper wird abgesaugt und getrocknet.
Ausbeute: 1,37 g (90,7 % der Theorie),
Schmelzpunkt: 204-207°C (Zers.)
$R_f$-Wert: 0,16 (Kieselgel; Methylenchlorid/Methanol = 9:1)
Analog Beispiel 9 kann folgende Verbindung hergestellt werden:

(1) N-[4-[[4-(4-Pyridyl)-piperazin-1-yl]carbonyl]piperidinyl]glycin-methylester-dihydrochlorid

Hergestellt aus N-tert.Butyloxycarbonyl-N-[4-[[4-(4-pyridyl)-piperazin-1-yl]carbonyl]piperidinyl]glycin-methylester.

Beispiel 10

3-[4-[4-(4-Pyridyl)-piperazin-1-yl]carbonylamino]piperidinopropionsäure-cyclohexylester-dihydrochlorid

Durch Suspension von 300 mg (0,7 mMol) 3-[4-[4-(4-Pyridyl)-piperazin-1-yl]carbonylamino]piperidinopropionsäure-dihydrochlorid in 20 ml Cyclohexanol leitet man während einer halben Stunde einen schwachen Strom von Salzsäuregas. Man läßt über Nacht bei Raumtemperatur stehen und erwärmt anschließend noch 2 Stunden lang auf Rüchflußtemperatur. Nach dem Abkühlen gießt man auf Ether und saugt den Nierderschlag ab.

Analog Beispiel 10 können folgende Verbindungen hergestellt werden:

(1) 3-[4-[4-(4-Pyridyl)-piperazin-1-yl]carbonylamino]piperidinopropionsäure-isobutylester-dihydrochlorid

Hergestellt aus 3-[4-[4-(4-Pyridyl)-piperazin-1-yl]carbonylamino]piperidinopropionsäure-dihydrochlorid und Isobutanol.

(2) [4-[4-(4-Pyridyl)-piperazino-1-yl]carbonylamino]piperidinoessigsäure-isobutylester-dihydrochlorid

Hergestellt aus [4-[4-(4-Pyridyl)-piperazin-1-yl]carbonylamino]piperidinoessigsäure und Isobutanol.

(3) [4-[4-(4-Pyridyl)-piperazino-1-yl]carbonylamino]piperidinoessigsäure-cyclohexlester-dihydrochlorid

Hergestellt aus [4-[4-(4-Pyridyl)-piperazin-1-yl]carbonylamino]piperidinoessigsäure und Cyclohexanol.

(4) [4-trans-[[4-(4-Pyridyl)-piperazin-1-yl]acetyl]amino]cyclohexancarbonsäurecyclohexylester-dihydrochlorid

Hergestellt aus [4-trans-[[4-(4-Pyridyl)-piperazin-1-yl]acetyl]amino]cyclohexancarbonsäure und Cyclohexanol.
$R_f$-Wert: 0,23 (Kieselgel; Methylenchlorid/Methanol/konz. Ammoniak = 9:1:0,1)
Massenspektrum: $M^+$ = 428

(5) [4-trans-[[4-(4-Pyridyl)-piperazin-1-yl]acetyl]amino]cyclohexancarbonsäure-isobutylester-dihydrochlorid

Hergestellt aus [4-trans-[[4-(4-Pyridyl)-piperazin-1-yl]acetyl]amino]cyclohexancarbonsäure und Isobutanol.
$R_f$-Wert: 0,22 (Kieselgel; Methylenchlorid/Methanol/konz. Ammoniak = 9:1:0,1)
Massenspektrum: $M^+$ = 402

Beispiel 11

3-[4-[4-(4-Pyridyl)-piperazin-1-yl]-carbonylamino]piperidinopropionsäure-pivaloyloxymethylester

Eine Mischung aus äquimolaren Teilen 3-[4-[4-(4-Pyridyl)-piperazin-1-yl]carbonylamino]piperidinopropionsäure, Pivalinsäurechlormethylester, Kaliumjodid und Kaliumcarbonat wird in Dimethylformamid bei Raumtemperatur während 2 Tagen gerührt. Anschließend wird in Wasser gegossen und mit Essigester extrahiert. Die vereinigten organischen Phasen werden getrocknet und unter Vakuum zur Trockne eingeengt. Der verbleibende Rückstand wird mittels Chromatographie über eine Kieselgel-Säule gereinigt.

Analog Beispiel 11 kann folgende Verbindung hergestellt werden:

(1) 3-[4-[4-(4-Pyridyl)-piperazin-1-yl]carbonylamino]piperidinopropionsäure-(1-ethyloxy)-carbonyloxyethylester

Hergestellt aus 3-[4-[4-(4-Pyridyl)-piperazin-1-yl]carbonylamino]piperidinopropionsäure und 1-Chlorethyl-ethylcarbonat.

Beispiel 12

[4-trans-[2S-[4-(4-Pyridyl)-piperazin-1-yl]propionyl]amino]cyclohexancarbonsäure-dihydrochlorid

Eine äquimolare Lösung von 4-trans-(L-Alanyl)-amino-cyclohexancarbonsäuremethylester-trifluoracetat und 4-Pyridyl-N,N-bis-(2-chlorethyl)amin-hydrochlorid wird zusammen mit 8 Mol N-Ethyl-diisopropylamin in Ethanol während 20 Stunden auf Rückflußtemperatur erhitzt. Anschließend wird die Lösung unter reduziertem Druck eingeengt und der Rückstand zwischen Wasser und Essigester verteilt. Der nach Trocknen und Einengen verbleibende Rückstand wird mittels Chromatographie gereinigt.

Analog Beispiel 12 werden folgende Verbindungen hergestellt:

(1) [4-trans-[2S-[4-(4-Pyridyl)-piperazin-1-yl]-(3-(4-methoxyphenyl)-propionyl)]amino]cyclohexancarbonsäuremethylesterdihydrochlorid

Hergestellt aus 4-trans-(O-Methyl-L-tyrosyl)-amino-cyclohexancarbonsäuremethylester-trifluoracetat, 4-Pyridyl-N,N-bis(2-chlorethyl)amin-hydrochlorid und N-Ethyl-diisopropylamin.

(2) N-[2S-[4-(4-Pyridyl)-piperazin-1-yl]-propionyl]-(piperidin-4-yloxy)-essigsäure-methylester-dihydrochlorid

Hergestellt aus N-(L-Alanyl)-4-piperidinyloxyessigsäuremethylester-trifluoracetat, 4-Pyridyl-N,N-bis-(2-chlorethyl)aminhydrochlorid und N-Ethyl-diisopropylamin.

(3) N-[2S-[4-(4-Pyridyl)-piperazin-1-yl]-(3-(4-methoxyphenyl)-propionyl)]-(piperidin-4-yloxy)-essigsäuremethylesterdihydrochlorid

Hergestellt aus N-(O-Methyl-L-tyrosyl)-4-piperidinyloxyessigsäuremethylester-trifluoracetat, 4-Pyridyl-N,N-bis(2-chlorethyl)amin-hydrochlorid und N-Ethyl-diisopropylamin.

Beispiel 13

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 14

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 15

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2.0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 16

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 17

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

Beispiel 18

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 0 abgefüllt.

## Patentansprüche

1. Piperazinderivate der allgemeinen Formel

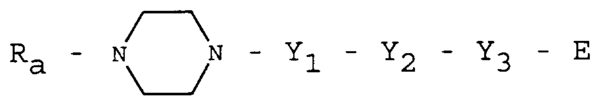

, (I)

in der
mit der Maßgabe, daß, wenn $R_a$ eine 4-Pyridylgruppe darstellt, nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO-$Gruppe,
$Y_2$ eine 1,3- oder 1,4-Phenylengruppe,
$Y_3$ eine-$CH_2CO-$, $-CH_2CH_2CO-$ oder $-OCH_2CO-$Gruppe und
E eine Hydroxy-, Methoxy- oder Ethoxygruppe oder

(b) $Y_1$ eine $-CH_2CO-$Gruppe,
$Y_2$ eine 3- oder 4-Piperidinylengruppe,
$Y_3$ eine -CO-, $-CH_2CO-$ oder $-OCH_2CO-$Gruppe und
E eine Hydroxy- oder Ethoxygruppe darstellen,

(c) $Y_1$ eine $-COCH_2-$Gruppe,
$Y_2$ eine 1,4-Phenylengruppe,
$Y_3$ eine $-OCH_2CO-$Gruppe und
E eine Hydroxy- oder tert.Butyloxygruppe darstellen,

$R_a$ eine Pyridylgruppe,
$Y_1$ eine -CO-, -CO-CO-, $-A_1-CO-$, $-CO-A_1-$, $-SO_2-A_2-$, $-A_2-SO_2-$, $-CO-A_1-CO-$, $-CO-NR_1-CO-$, $-CO-NR_1-A_2-$, $-CO-NR_1-A_2-CO-$ oder $-CO-A_2-NR_1-CO-$Gruppe, in denen
$R_1$ ein Wasserstoffatom, eine $C_{1-5}$-Alkyl-, Aryl- oder Aryl-$C_{1-3}$-alkylgruppe,
$A_1$ eine gegebenenfalls durch eine $C_{1-5}$-Alkyl-, Cyclohexyl-$C_{1-3}$-alkyl-, Aryl- oder Aryl-$C_{1-3}$-alkylgruppe oder auch durch eine $R_1O$-Gruppe, sofern diese nicht in $\alpha$-Stellung zu einem Stickstoffatom steht, substituierte n-$C_{1-5}$-Alkylengruppe und
$A_2$ eine gegebenenfalls durch eine $C_{1-5}$-Alkyl-, Aryl- oder Aryl-$C_{1-3}$-alkylgruppe substituierte n-$C_{1-4}$-Alkylengruppe darstellen,
$Y_2$ eine Phenylen-, Cyclohexylen- oder Pyridinylengruppe, eine 3-Piperidinylen-, 4-Piperidinylen- oder 1,4-Piperazinylengruppe, in denen jeweils eine oder zwei zu einem Stickstoffatom benachbarte Methylengruppen durch eine Carbonylgruppe ersetzt sein können, eine $-NR_1-B-$ oder -O-B-Gruppe, wobei die Verknüpfung mit der $Y_1$-Gruppe über das Stickstoffatom der $-NR_1$-Gruppe oder über das Sauerstoffatom der -O-B-Gruppe erfolgt, in denen
$R_1$ wie eingangs definiert ist und
B eine Phenylen-, Cyclohexylen-, Piperidinylen- oder Pyridinylengruppe darstellt, wobei die Verknüpfung der Piperidinylengruppe jeweils über die 3- oder 4-Stellung mit dem Rest $-NR_1-$ oder mit dem Sauerstoffatom erfolgt und in der zusätzlich eine zu einem Stickstoffatom benachbarte Methylengruppe durch eine Carbonylgruppe ersetzt sein kann,
$Y_3$ eine -CO-, $-A_2-CO-$, $-CH_2-CH(NHR_2)-CO-$, $-NR_2-A_3-CO-$, $-O-A_3-CO-$ oder $-CO-A_3-CO-$Gruppe, in denen
$R_1$ und $A_2$ wie eingangs definiert sind,
$A_3$ eine gegebenenfalls durch eine $C_{1-5}$-Alkyl-, Aryl- oder Aryl-$C_{1-3}$-alkylgruppe substituierte n-$C_{1-3}$-Alkylengruppe und
$R_2$ ein Wasserstoffatom, eine $C_{1-5}$-Alkyl-, Aryl-$C_{1-3}$-alkyl-, Aryl-, $C_{1-5}$-Alkoxycarbonyl-, $C_{1-5}$-Alkanoyl-, $C_{1-5}$-Alkylsulfonyl-, Aryl-$C_{1-3}$-alkylsulfonyl- oder Arylsulfonylgruppe, eine gegebenenfalls durch eine Aryl- oder Aryl-$C_{1-3}$-alkylgruppe substituierte Formylgruppe darstellen sowie die Verknüpfung der $-A_2-CO-$Gruppe über den Rest $A_2$, der $-NR_2-A_3-CO-$Gruppe über die $-NR_2-$Gruppe und der $-O-A_3-CO-$Gruppe über das Sauerstoffatom mit dem Rest $Y_2$ erfolgt, wobei jedoch eine $-NR_2-$ oder $-O-A_3-CO-$Gruppe nicht mit einem Stickstoffatom des Restes $Y_2$ verknüpft sein kann,
und E eine Hydroxygruppe, eine Alkoxygruppe mit 1 bis 6 Kohlenstoffatomen, eine Phenylalkoxygruppe, in der der Alkoxyteil 1 bis 3 Kohlenstoffatome enthalten kann, eine Cycloalkoxygruppe mit 3 bis 9 Kohlenstoffatomen, in welcher der Cycloalkylteil mit 5 bis 8 Kohlenstoffatomen zusätzlich durch ein oder zwei Alkylgruppen mit jeweils 1 bis 3 Kohlenstoffatomen substituiert sein kann, eine Cycloalkoxygruppe mit 5 bis 8 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe, in denen der Alkyl- und Alkoxyteil jeweils 1 bis 3 Kohlenstoffatomen enthalten kann, oder durch eine Alkanoylgruppe mit 2 bis 6 Kohlenstoffatomen substituierte Iminogruppe ersetzt ist und der Cycloalkylteil zusätzlich durch ein oder zwei Alkylgruppen mit jeweils 1 bis 3 Kohlenstoffatomen substituiert sein kann, eine Cycloalkenyloxygruppe, in der der Cycloalkenylteil 4 bis 7 Kohlenstoffatome enthalten kann, eine Alkenyloxy-, Phenylalkenyloxy-, Alkinyloxy- oder Phenylalkinyloxygruppe mit der Maßgabe, daß keine Bindung an das Sauerstoffatom von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkoxygruppe, in der der Cycloalkylteil 3 bis 8 Kohlenstoffatome und der Alkoxyteil 1 bis 3 Kohlenstoffatome enthalten kann, eine Bicycloalkoxygruppe mit insgesamt 8 bis 10 Kohlenstoffatomen, die im Bicycloalkylteil zusätzlich durch ein oder zwei Alkylgruppen mit jeweils 1 bis 3 Kohlenstoffatomen substituiert sein kann, eine 1,3-Dihydro-3-oxo-1-isobenzfuranyloxygruppe oder eine $R_5$-CO-O-($R_3CR_4$)-O-Gruppe, in der

$R_3$ ein Wasserstoffatom, eine $C_{1-6}$-Alkyl-, $C_{3-7}$-Cycloalkyl- oder Phenylgruppe,

$R_4$ ein Wasserstoffatom oder eine $C_{1-6}$-Alkylgruppe und $R_5$ eine $C_{1-5}$-Alkyl-, $C_{1-5}$-Alkoxy-, $C_{5-7}$-Cycloalkyl- oder $C_{5-7}$-Cycloalkoxygruppe darstellen,

oder E eine $\alpha$-Aminogruppe einer natürlichen Aminosäure und deren Ester bedeuten, wobei

unter dem bei der Definition der vorstehenden Resten erwähnten Ausdrücken "eine Arylgruppe," "eine Phenylgruppe" oder "eine Phenylengruppe" jeweils insbesondere eine gegebenenfalls durch Fluor-, Chlor-, Brom- oder Jodatome, durch Alkyl-, Trifluormethyl-, Nitro-, Amino-, Alkylamino-, Dialkylamino-, Alkanoylamino-, Hydroxy-, Alkoxy-, Carboxy-, Alkoxycarbonyl-, Hydroxycarbonylalkoxy-, Alkoxycarbonylalkoxy-, Aminocarbonyl-, Alkylaminocarbonyl- oder Dialkylaminocarbonylgruppen mono-, di- oder trisubstituierte Phenyl- oder Phenylengruppe, wobei die Substituenten gleich oder verschieden sein können und die vorstehend erwähnten Alkyl- und Alkoxyteile jeweils 1 bis 3 Kohlenstoffatome enthalten können, und

unter den Estern einer natürlichen $\alpha$-Aminogruppe deren $C_{1-6}$-Alkyl-, $C_{2-6}$-Alkenyl-, $C_{5-7}$-Cycloalkyl-, Phenyl- oder Phenyl-$C_{1-3}$-alkylester zu verstehen ist,

deren Tautomere, deren Stereoisomere, einschließlich ihrer Gemische, und deren Salze.

2. Piperazinderivate der allgemeinen Formel I gemäß Anspruch 1, in denen mit der Maßgabe, daß, wenn $R_a$ eine 4-Pyridylgruppe darstellt, nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO-$-Gruppe,
$Y_2$ eine 1,3- oder 1,4-Phenylengruppe,
$Y_3$ eine-$CH_2CO-$, $-CH_2CH_2CO-$ oder $-OCH_2CO-$-Gruppe und
E eine Hydroxy-, Methoxy- oder Ethoxygruppe oder

(b) $Y_1$ eine $-CH_2CO-$-Gruppe,
$Y_2$ eine 3- oder 4-Piperidinylengruppe,
$Y_3$ eine -CO-, $-CH_2CO-$ oder $-OCH_2CO-$-Gruppe und
E eine Hydroxy- oder Ethoxygruppe darstellen,

(c) $Y_1$ eine $-COCH_2-$-Gruppe,
$Y_2$ eine 1,4-Phenylengruppe,
$Y_3$ eine $-OCH_2CO-$-Gruppe und
E eine Hydroxy- oder tert.Butyloxygruppe darstellen,

$R_a$ eine 3- oder 4-Pyridylgruppe,

$Y_1$ eine -CO-, -CO-CO-, $-A_1$-CO-, -CO-$A_1$- oder -CO-$A_1$-CO-Gruppe, in denen

$A_1$ eine gegebenenfalls durch eine $C_{1-5}$-Alkyl-, Phenyl- oder Phenyl-$C_{1-3}$-alkylgruppe substituierte n-$C_{1-5}$-Alkylengruppe darstellt, wobei die Phenylkerne der vorstehend erwähnten Phenyl- und Phenylalkylgruppen jeweils zusätzlich durch eine Hydroxy- oder Methoxygruppe substituiert sein können,

$Y_2$ eine 1,3-Phenylen-, 1,4-Phenylen-, 3-Piperidinylen-, 4-Piperidinylen-, 1,4-Piperazinylen- oder $-NR_1$-B-Gruppe, wobei die Verknüpfung mit der $Y_1$-Gruppe über das Stickstoffatom der $-NR_1$-Gruppe erfolgt und

$R_1$ ein Wasserstoffatom, eine $C_{1-5}$-Alkyl-, Phenyl- oder Phenyl-$C_{1-3}$-alkylgruppe und

B eine 1,3-Phenylen-, 1,4-Phenylen-, 1,3-Cyclohexylen-, 1,4-Cyclohexylen-, 3-Piperidinylen- oder 4-Piperidinylengruppe darstellen,

$Y_3$ eine -CO-, $-A_2$-CO-, $-NR_2$-$A_3$-CO- oder -O-$A_3$-CO-Gruppe, in denen

$A_2$ eine gegebenenfalls durch eine $C_{1-5}$-Alkyl-, Phenyl- oder Phenyl-$C_{1-3}$-alkylgruppe substituierte n-$C_{1-4}$-

Alkylengruppe,

$A_3$ eine gegebenenfalls durch eine $C_{1-5}$-Alkyl-, Phenyl- oder Phenyl-$C_{1-3}$-alkylgruppe substituierte n-$C_{1-3}$-Alkylengruppe und

$R_2$ ein Wasserstoffatom, eine $C_{1-5}$-Alkyl-, Phenyl-$C_{1-3}$-alkyl-, Phenyl-, $C_{1-5}$-Alkoxycarbonyl- oder $C_{1-5}$-Alkanoylgruppe darstellen sowie die Verknüpfung der -$A_2$-CO-Gruppe über den Rest $A_2$, der -$NR_2$-$A_3$-CO-Gruppe über die -$NR_2$-Gruppe und der -O-$A_3$-CO-Gruppe über das Sauerstoffatom mit dem Rest $Y_2$ erfolgt, wobei jedoch eine -$NR_2$- oder -O-$A_3$-CO-Gruppe nicht mit einem Stickstoffatom des Restes $Y_2$ verknüpft sein kann,

und E eine Hydroxygruppe, eine Alkoxygruppe mit 1 bis 6 Kohlenstoffatomen, eine Phenylalkoxygruppe, in der der Alkoxyteil 1 bis 3 Kohlenstoffatome enthalten kann, eine Cycloalkoxygruppe mit 4 bis 7 Kohlenstoffatomen oder eine $R_5$-CO-O-($R_3CR_4$)-O-Gruppe, in der

$R_3$ ein Wasserstoffatom, eine $C_{1-6}$-Alkyl-, $C_{3-7}$-Cycloalkyl- oder Phenylgruppe,

$R_4$ ein Wasserstoffatom oder eine $C_{1-6}$-Alkylgruppe und $R_5$ eine $C_{1-5}$-Alkyl-, $C_{1-5}$-Alkoxy-, $C_{5-7}$-Cycloalkyl- oder $C_{5-7}$-Cycloalkoxygruppe darstellen,

oder E eine $\alpha$-Aminogruppe einer natürlichen Aminosäure oder deren Estern bedeuten,

deren Tautomere, deren Stereoisomere, einschließlich ihrer Gemische, und deren Salze.

**3.** Piperazinderivate der allgemeinen Formel I gemäß Anspruch 1, in denen mit der Maßgabe, daß nicht gleichzeitig

(a) $Y_1$ eine -$CH_2$CO-, -CH($CH_3$)CO-, -C($CH_3$)$_2$CO-, -$CH_2CH_2$CO- oder -$CH_2$CH($CH_3$)CO-Gruppe,
$Y_2$ eine 1,3- oder 1,4-Phenylengruppe,
$Y_3$ eine-$CH_2$CO-, -$CH_2CH_2$CO- oder -O$CH_2$CO-Gruppe und
E eine Hydroxy-, Methoxy- oder Ethoxygruppe oder

(b) $Y_1$ eine -$CH_2$CO-Gruppe,
$Y_2$ eine 3- oder 4-Piperidinylengruppe,
$Y_3$ eine -CO-, -$CH_2$CO- oder -O$CH_2$CO-Gruppe und
E eine Hydroxy- oder Ethoxygruppe darstellen,

(c) $Y_1$ eine -CO$CH_2$-Gruppe,
$Y_2$ eine 1,4-Phenylengruppe,
$Y_3$ eine -O$CH_2$CO-Gruppe und
E eine Hydroxy- oder tert.Butyloxygruppe darstellen,

$R_a$ eine 4-Pyridylgruppe,

$Y_1$ eine -CO-, -CO-CO-, -$A_1$-CO-, -CO-$A_1$- oder -CO-$A_1$-CO-Gruppe, in denen

$A_1$ eine gegebenenfalls durch eine $C_{1-5}$-Alkyl-, Phenyl- oder Phenyl-$C_{1-2}$-alkylgruppe substituierte n-$C_{1-5}$-Alkylengruppe darstellt, wobei die Phenylkerne der vorstehend erwähnten Phenyl- und Phenylalkylgruppen jeweils zusätzlich durch eine Hydroxy- oder Methoxygruppe substituiert sein können,

$Y_2$ eine 1,4-Phenylen-, 4-Piperidinylen-, 1,4-Piperazinylen- oder -$NR_1$-B-Gruppe, wobei die Verknüpfung mit der $Y_1$-Gruppe über das Stickstoffatom der -$NR_1$-Gruppe erfolgt und

$R_1$ ein Wasserstoffatom, eine $C_{1-5}$-Alkyl-, Phenyl- oder Phenyl-$C_{1-2}$-alkylgruppe und

B eine 1,3-Phenylen-, 1,4-Phenylen-, 1,3-Cyclohexylen-, 1,4-Cyclohexylen-, 3-Piperidinylen- oder 4-Piperidinylengruppe darstellen,

$Y_3$ eine -CO-, -$A_2$-CO-, -$NR_2$-$A_3$-CO- oder -O-$A_3$-CO-Gruppe, in denen

$A_2$ eine gegebenenfalls durch eine $C_{1-3}$-Alkyl-, Phenyl- oder Phenyl-$C_{1-2}$-alkylgruppe substituierte n-$C_{1-4}$-Alkylengruppe,

$A_3$ eine gegebenenfalls durch eine $C_{1-5}$-Alkyl-, Phenyl- oder Phenyl-$C_{1-3}$-alkylgruppe substituierte n-$C_{1-3}$-Alkylengruppe und

$R_2$ ein Wasserstoffatom, eine $C_{1-3}$-Alkyl-, $C_{1-5}$-Alkoxycarbonyl- oder $C_{1-3}$-Alkanoylgruppe darstellen sowie die Verknüpfung der -$A_2$-CO-Gruppe über den Rest $A_2$, der -$NR_2$-$A_3$-CO-Gruppe über die -$NR_2$-Gruppe und der -O-$A_3$-CO-Gruppe über das Sauerstoffatom mit dem Rest $Y_2$ erfolgt, wobei jedoch eine -$NR_2$- oder -O-$A_3$-CO-Gruppe nicht mit einem Stickstoffatom des Restes $Y_2$ verknüpft sein kann,

und E eine Hydroxygruppe, eine Alkoxygruppe mit 1 bis 5 Kohlenstoffatomen, eine Cycloalkoxygruppe mit 5 bis 7 Kohlenstoffatomen oder eine $R_5$-CO-O-($R_3CR_4$)-O-Gruppe, in der

$R_3$ ein Wasserstoffatom, eine $C_{1-3}$-Alkyl- oder $C_{5-7}$-Cycloalkylgruppe,

$R_4$ ein Wasserstoffatom und

$R_5$ eine $C_{1-5}$-Alkyl- oder $C_{1-3}$-Alkoxygruppe darstellen,

oder E eine $\alpha$-Aminogruppe einer natürlichen Aminosäure oder deren Estern mit einem $C_{1-6}$-Alkanol oder Benzyl-

alkohol bedeuten,
deren Tautomere, deren Stereoisomere, einschließlich ihrer Gemische, und deren Salze.

4. Piperazinderivate der allgemeinen Formel I gemäß Anspruch 1, in denen mit der Maßgabe, daß nicht gleichzeitig

(a) $Y_1$ eine $-CH_2CO-$, $-CH(CH_3)CO-$, $-C(CH_3)_2CO-$, $-CH_2CH_2CO-$ oder $-CH_2CH(CH_3)CO-$Gruppe,
$Y_2$ eine 1,3- oder 1,4-Phenylengruppe,
$Y_3$ eine-$CH_2CO-$, $-CH_2CH_2CO-$ oder $-OCH_2CO-$Gruppe und
E eine Hydroxy-, Methoxy- oder Ethoxygruppe oder

(b) $Y_1$ eine $-CH_2CO-$Gruppe,
$Y_2$ eine 4-Piperidinylengruppe,
$Y_3$ eine -CO-, $-CH_2CO-$ oder $-OCH_2CO-$Gruppe und
E eine Hydroxy- oder Ethoxygruppe darstellen,

(c) $Y_1$ eine $-COCH_2-$Gruppe,
$Y_2$ eine 1,4-Phenylengruppe,
$Y_3$ eine $-OCH_2CO-$Gruppe und
E eine Hydroxy- oder tert.Butyloxygruppe darstellen,

$R_a$ eine 4-Pyridylgruppe,
$Y_1$ eine -CO-, -COCO-, $-A_1-CO-$, $-CO-A_1-$ oder $-CO-CH_2-CO-$Gruppe, in denen
$A_1$ eine gegebenenfalls durch eine Methyl- oder Methoxyphenylgruppe substituierte n-$C_{1-4}$-Alkylengruppe darstellt,
$Y_2$ eine 1,4-Phenylen-, 4-Piperidinylen-, 1,4-Piperazinylen- oder $-NR_1-B-$Gruppe, wobei die Verknüpfung mit der $Y_1$-Gruppe über das Stickstoffatom der $-NR_1-$Gruppe erfolgt und
$R_1$ ein Wasserstoffatom und
B eine 1,3-Phenylen-, 1,4-Phenylen-, 1,3-Cyclohexylen-, 1,4-Cyclohexylen- oder 4-Piperidinylengruppe darstellen,
$Y_3$ eine -CO-, $-A_2-CO-$, $-NR_2-A_3-CO-$ oder $-O-A_3-CO-$Gruppe, in denen
$A_2$ eine n-$C_{1-3}$-Alkylengruppe,
$A_3$ eine $C_{1-2}$-Alkylengruppe und
$R_2$ ein Wasserstoffatom, eine Methyl-, Benzyl-, Phenylethyl- oder Acetylgruppe darstellen sowie die Verknüpfung der $-A_2-CO-$Gruppe über den Rest $A_2$, der $-NR_2-A_3-CO-$Gruppe über die $-NR_2-$Gruppe und der $-O-A_3-CO-$Gruppe über das Sauerstoffatom mit dem Rest $Y_2$ erfolgt, wobei jedoch eine $-NR_2-$ oder $-O-A_3-CO-$Gruppe nicht mit einem Stickstoffatom des Restes $Y_2$ verknüpft sein kann,
und E eine Hydroxygruppe, eine Alkoxygruppe mit 1 bis 4 Kohlenstoffatomen, eine Cycloalkoxygruppe mit 5 bis 7 Kohlenstoffatomen oder eine $R_5-CO-O-(R_3CR_4)-O-$Gruppe, in der
$R_3$ ein Wasserstoffatom oder eine $C_{1-3}$-Alkylgruppe,
$R_4$ ein Wasserstoffatom und
$R_5$ eine $C_{1-5}$-Alkyl- oder $C_{1-3}$-Alkoxygruppe darstellen,
oder E eine Glycinylgruppe oder deren Methylester bedeuten,
deren Tautomere, deren Stereoisomere, einschließlich ihrer Gemische, und deren Salze.

5. Folgende Piperazinderivate der allgemeinen Formel I gemäß Anspruch 1:

(a) [4-trans-[3-[4-(4-Pyridyl)-piperazin-1-yl]propionyl]amino]cyclohexancarbonsäure,

(b) 3-[4-trans-[4-(4-Pyridyl)-piperazin-1-yl]carbonylamino]cyclohexylpropionsäure,

(c) 3-[4-trans-[4-(4-Pyridyl)-piperazin-1-yl]malonylamino]cyclohexylcarbonsäure,

(d) 3-[4-[4-(4-Pyridyl)-piperazin-1-yl]carbonylamino]piperidinopropionsäure,

(e) [4-[4-(4-Pyridyl)-piperazin-1-yl]carbonylamino]piperidinoessigsäure,

(f) [4-trans-[3-[4-(4-Pyridyl)-piperazin-1-yl]propionyl]amino]cyclohexancarbonsäure-methylester,

(g) 3-[4-trans-[4-(4-Pyridyl)-piperazin-1-yl]carbonylamino]cyclohexylpropionsäure-methylester,

(h) [4-trans-[4-(4-Pyridyl)-piperazin-1-yl]malonylamino]cyclohexylcarbonsäure-methylester,

(i) 4-[[4-(4-Pyridyl)-piperazin-1-yl]carbonylamino]piperidinoessigsäure-methylester,

(j) [4-trans-[[4-(4-Pyridyl)-piperazin-1-yl]acetyl]amino]cyclohexancarbonsäurecyclohexylester,

(k) [4-trans-[[4-(4-Pyridyl)-piperazin-1-yl]acetyl]amino]cyclohexancarbonsäure-isobutylester,

deren Tautomere und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Verbindungen der allgemeinen Formel I gemäß den Ansprüchen 1 bis 6, dadurch gekennzeichnet, daß

a. eine Verbindung der allgemeinen Formel

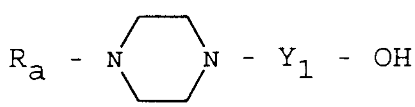

, (II)

in der
$R_a$ und $Y_1$ wie eingangs definiert sind, oder deren reaktionsfähigen Derivaten mit einer Verbindung der allgemeinen Formel

$$H - Y_2' - Y_3 - E' \quad ,(III)$$

in der
$Y_3$ wie in den Ansprüchen 1 bis 5 definiert ist,
$Y_2'$ mit Ausnahme der Phenylengruppe die für $Y_2$ in den Ansprüchen 1 bis 5 erwähnten Bedeutungen aufweist und
E' eine $C_{1-6}$-Alkoxy-, Phenyl-$C_{1-3}$-alkoxy- oder $C_{5-7}$-Cycloalkoxygruppe darstellt, umgesetzt wird oder

b. zur Herstellung einer Verbindung der allgemeinen Formel I, in der mindestens einer der Reste $R_2$ oder E ein reaktionsfähiges Wasserstoffatom mit der Maßgabe enthalten muß, daß E mit Ausnahme der $R_5$-CO-O-($R_3CR_4$)-O-Gruppe die für E in den Ansprüchen 1 bis 5 erwähnten Bedeutungen aufweist, eine Verbindung der allgemeinen Formel

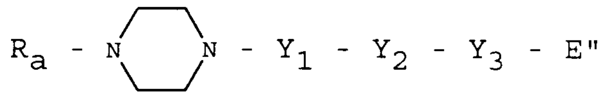 , (IV)

in der
$R_a$ und $Y_1$ bis $Y_3$ wie in den Ansprüchen 1 bis 5 definiert sind und
E" eine Hydroxygruppe oder zusammen mit der benachbarten Carbonylgruppe des Restes $Y_3$ eine mittels Hydrolyse, Behandeln mit einer Säure oder Base, Thermolyse oder Hydrogenolyse abspaltbaren Schutzrest in eine Carboxylgruppe überführbare Gruppe bedeutet, wobei jedoch mindestens einer der Reste $NR_2$ oder E" einen abspaltbaren Rest enthalten muß,
in eine Verbindung der allgemeinen Formel I, in der mindestens einer der Reste $R_2$ oder E ein reaktionsfähiges Wasserstoffatom mit der Maßgabe enthalten muß, daß E mit Ausnahme der $R_5$-CO-O-($R_3CR_4$)-O-Gruppe die für E in den Ansprüchen 1 bis 5 erwähnten Bedeutungen aufweist, übergeführt wird oder

c. zur Herstellung einer Verbindung der allgemeinen Formel I, in der $Y_2$ mit Ausnahme der Phenylengruppe wie in den Ansprüchen 1 bis 5 erwähnt definiert ist und $Y_3$ eine -$A_2$-CO- Gruppe, in der $A_2$ eine gegebenenfalls durch eine $C_{1-5}$-Alkyl-, Phenyl- oder Phenyl-$C_{1-3}$-alkylgruppe substituierte n-$C_{2-4}$-Alkenylengruppe bedeutet, darstellen, eine Verbindung der allgemeinen Formel

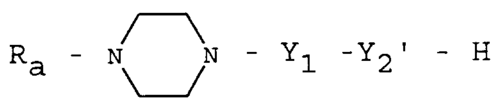 , (V)

in der
$R_a$ und $Y_1$ wie in den Ansprüchen 1 bis 5 definiert sind und
$Y_2$' mit Ausnahme der Phenylen-, 3-Piperidinylen- oder 4-Piperidinylengruppe die für $Y_2$ in den Ansprüchen 1 bis 5 erwähnten Bedeutungen aufweist, mit einer Verbindung der allgemeinen Formel

$$A_2' - CO - E \quad ,(VI)$$

in der
E wie in den Ansprüchen 1 bis 5 definiert ist und
$A_2$' eine gegebenenfalls durch eine $C_{1-5}$-Alkyl-, Phenyl- oder Phenyl-$C_{1-3}$-alkylgruppe substituierte n-$C_{2-4}$-Alkenylengruppe darstellt, umgesetzt wird oder

d. eine Verbindung der allgemeinen Formel

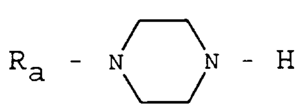 , (VII)

in der
Ra wie in den Ansprüchen 1 bis 5 definiert ist, mit einer Verbindung der allgemeinen Formel

$$Z_1 - Y_1 - Y_2 - Y_3 - E \quad ,(VIII)$$

in der
$Y_1$, $Y_2$, $Y_3$ und E wie in den Ansprüchen 1 bis 5 definiert sind und
$Z_1$ eine nukleophile Austrittsgruppe oder auch, wenn $Y_1$ eine Carbonylgruppe darstellt,
$Z_1$ zusammen mit $R_1$ eine weitere Kohlenstoff-Stickstoff-Bindung bedeutet, umgesetzt wird oder

e. zur Herstellung einer Verbindung der allgemeinen Formel I, in der E eine $C_{1-6}$-Alkoxy-, Phenyl-$C_{1-3}$-alkoxy-, $C_{5-7}$-Cycloalkoxy- oder $R_5$-CO-O-($R_3CR_4$)-O-Gruppe darstellt, eine Verbindung der allgemeinen Formel

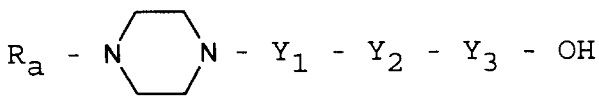

, (IX)

in der
$R_a$ und $Y_1$ bis $Y_3$ wie in den Ansprüchen 1 bis 5 definiert sind, mit einer Verbindung der allgemeinen Formel

$$HO - R_b \qquad ,(X)$$

oder mit einer Verbindung der allgemein Formel

$$Z_2 - R_c \qquad ,(XI)$$

in denen
$R_b$ eine $C_{1-6}$-Alkyl-, Phenyl-$C_{1-3}$-alkyl- oder $C_{5-7}$-Cycloalkylgruppe,
$R_c$ eine eine $C_{1-6}$-Alkyl-, Phenyl-$C_{1-3}$-alkyl-, $C_{5-7}$-Cycloalkyl- oder $R_5$-CO-O-($R_3CR_4$)-Gruppe, in der $R_3$, $R_4$ und $R_5$ wie in den Ansprüchen 1 bis 5 definiert sind, und
$Z_2$ eine Austrittsgruppe darstellen, umgesetzt wird oder

f. eine Verbindung der allgemeinen Formel

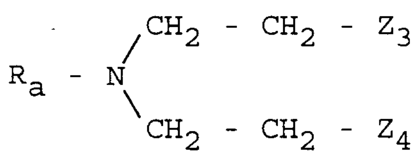

, (XII)

in der
$R_a$ wie wie in den Ansprüchen 1 bis 5 definiert ist,
$Z_3$ und $Z_4$, die gleich oder verschieden sein können, nukleophile Austrittsgruppen bedeuten, mit einer Verbindung der allgemeinen Formel

$$NH_2 - Y_1 - Y_2 - Y_3 - E \qquad ,(XIII)$$

in der
E, $Y_2$ und $Y_3$ wie wie in den Ansprüchen 1 bis 5 definiert sind und
$Y_1$ eine -$A_1$-CO- oder -$A_2$-$SO_2$-gruppe darstellt, wobei $A_1$ und $A_2$ wie wie in den Ansprüchen 1 bis 5 definiert sind, umgesetzt wird und
und erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträgliche Salze übergeführt wird.